# EUROPÄISCHE PATENTSCHRIFT

(11) **EP 4 337 443 B1**
(45) Veröffentlichungstag und Bekanntmachung des Hinweises auf die Patenterteilung: **30.04.2025**
(21) Anmeldenummer: 22708288.0
(22) Anmeldetag: 07.01.2022
(51) Int. Cl.: B29C 44/34, A61L 31/14

(54) **AUXETISCHE STEG- ODER FELDSTRUKTUR SOWIE VERWENDUNG**
AUXETIC WEB STRUCTURE OR FIELD STRUCTURE, AND USE
STRUCTURE DE BANDE AUXÉTIQUE OU STRUCTURE DE CHAMP, ET UTILISATION

(30) Priorität: 20.02.2021 DE 102021104058; 20.02.2021 DE 102021104057
(43) Veröffentlichungstag der Anmeldung: 20.03.2024
(73) Patentinhaber: Christian-Albrechts-Universität zu Kiel, 24118 Kiel (DE)
(72) Erfinder: CURTIS, Sabrina M., Frederick, Maryland 21701 (US); DENGIZ, Duygu, 21465 Reinbek (DE); QUANDT, Eckhard, 24226 Heikendorf (DE)
(86) Internationale Anmeldenummer: PCT/DE2022/100001
(87) Internationale Veröffentlichungsnummer: WO 2022/174856

(56) Entgegenhaltungen:
- US-A1- 2014 117 165
- US-A1- 2020 144 431

## Beschreibung

Die Erfindung betrifft eine auxetische Steg- oder Feldstruktur aufweisend Inselstrukturflächen und Verbindungen zwischen den einzelnen Inselstrukturflächen, wobei offene Räume zwischen den einzelnen Inselstrukturflächen vorliegen und die Verbindungen mit den Inselstrukturflächen eine Steg- oder Feldstruktur ausbilden und die Verbindungen dehnbar ausgebildet sind.

Derartige Strukturen können auch als auxetische metamechanische Materialien mit verbesserter Dehnbarkeit und Kompressibilität bezeichnet werden.

Drei aktuelle Übersichtsarbeiten, nämlich:
- Prawoto, Yunan. "Seeing auxetic materials from the mechanics point of view: a structural review on the negative Poisson's ratio." Computational Materials Science 58 (2012): 140-153;
- Ren, Xin, et al. "Auxetic metamaterials and structures: a review." Smart materials and structures 27.2 (2018): 023001.
- Saxena, Krishna Kumar, Raj Das, and Emilio P. Calius. "Three decades of auxetics researchmaterials with negative Poisson's ratio: a review." Advanced Engineering Materials 18.11 (2016): 1847-1870
   und
- Wang, Zhenwei, et al. "Progress in Auxetic Mechanical Metamaterials: Structures, Characteristics, Manufacturing Methods, and Applications." Advanced Engineering Materials 22.10 (2020): 2000312
fassen die umfangreichen Fortschritte bei auxetischen Strukturen in den letzten drei Jahrzehnten aus über 300 Forschungsarbeiten in Bezug auf Modellierung, Herstellung und Charakterisierung von auxetischen Strukturen zusammen.

Die Verwendung dehnbarer Verbindungen zur Verbesserung der mechanischen Dehnungseigenschaften von auxetischen Strukturen wird in diesen Übersichtsarbeiten nicht erwähnt.

Die Druckschrift US 2018/0311833A1 beschreibt in "Non-planar shearing auxetic structures, devices, and methods" auxetische Strukturen als "A non-planar shearing auxetic structure, comprising each unit cell is defined by a plurality of elements and by an internal angle between two of the plurality of elements that are connected together by a pivot joint", wobei das dort offenbarte und beschriebene Drehgelenk im Prinzip der Ort der modifizierten dehnbaren Verbindung ist, die auch hier offenbart ist.

Hassanin, Hany et al. offenbaren in "4D printing of NiTi auxetic structure with improved ballistic performance" Micromachines 11.8 (2020): 745 die Entwicklung einer NiTi-Struktur mit einem negativen Poissonverhältnis mit Superelasitizität/Formgedächtnis für verbesserte ballistische Anwendungen. Für die Herstellung der optimierten auxetischen NiTi-Struktur wird ein 4D-Druckprozess genutzt.

Das einzige auxetische Design, das den heutzutage in der dehnbaren Elektronik verwendeten Verbindungsgeometrien ähnlich zu sein scheint, ist das rotachirale Design. Es ähnelt dem "2D wavy network constructed with horseshoe building blocks, configured into a triangle lattice geometry", vorgeschlagen von K-I Jang et al. in "Soft network composite materials with deterministic and bio-inspired designs" Nature Communications 6, 6566 (2015). Die Autoren stellen fest, dass bei geringen Dehnungen ein negativer Effekt der Poissonzahl der Struktur zu beobachten ist, jedoch wird das auxetische Verhalten des dehnbaren Netzes mit Dreiecksgittergeometrie nicht untersucht. Bei der quadratischen Gittergeometrie wird in dieser Druckschrift keine negative Poisson-Zahl festgestellt. Darüber hinaus bieten die vorgeschlagenen wellenförmigen Maschendesigns keinen großen Inselraum, der für eine mögliche Funktionalisierung elektronische Komponenten zur Verfügung steht.

Elektronische 2D-Dünnschichtgeräte, die als Kontaktstelle zu biologischen komplexen 3D-Oberflächen dienen sollen, müssen in der Lage sein, großen makroskopischen Belastungen standzuhalten, ohne die funktionellen Komponenten des Geräts zu beeinträchtigen. Auf dem Gebiet der flexiblen und dehnbaren Elektronik wurden zahlreiche Lösungen für dieses Problem gefunden, indem die Geometrie der gesamten elektronischen Schaltung neu konfiguriert wurde. Eine Lösung besteht darin, die elektronische Schaltung in ein wellenförmiges 2D-Dünnfilm-Metallnetz zu verwandeln. Eine andere beliebte Technik ist die "Insel-Brücke", bei der eine Reihe periodischer Inseln strukturiert wird, die in der Mitte durch dehnbare Verbindungen verbunden sind. Die Spannungen auf den Inseln bleiben auch bei Verformung gering, da sich die Spannungen und Dehnungen auf die dehnbaren Verbindungen konzentrieren. Beispiele für solche Verbindungen, die aus dem Stand der Technik bekannt sind, sind Serpentinen, fraktale Muster, 2D-Wellengitter und neuerdings auch archimedische Spiralstrukturen, die im Zentrum der Inseln miteinander verbunden sind. Die "Insel-Brücken"-Strukturen werden nach dem Stand der Technik so konstruiert, dass sie sich unter biaxialer Belastung ausdehnen. Diese Lösungen ermöglichen viele beeindruckende Geräte. Es gibt jedoch einige Einschränkungen für traditionelle dehnbare Designs, einschließlich einer positiven (oder Null) Poissonzahl. Zudem ist es in der Regel erforderlich, dass herkömmliche dehnbare Elektronik auf einem elastomeren Substrat integriert wird, um die elastische Erholung zu fördern. Es besteht die Notwendigkeit 2D-Elektronikbauteile in freistehende strukturelle Geometrien zu konfigurieren, die es dem Bauteil ermöglichen, große makroskopische Dehnungen elastisch zu erreichen, sich konform in 3D-Oberflächen zu integrieren und eine hohe Flächendichte für funktionale elektronische Komponenten zu erhalten.

In der Druckschrift US 8°552°299 B2 werden elektronische Schaltungen gezeigt, die auf dem Insel-Brücken-Layout basieren, das aus einer Anordnung von periodischen Inseln besteht, die durch Serpentinen verbunden sind. Die dargestellten Strukturen haben keine auxetischen Eigenschaften und weisen zudem eine Poisson-Zahl von Null oder positiv auf. In einigen Fällen imitieren die hergestellten Geräte ein ähnliches mechanisches Verhalten wie auxetische Strukturen (d.h. die Fähigkeit, sich über eine kuppelförmige Krümmung zu biegen, Ausdehnung unter biaxialer (x- und y-Richtung) Belastung). In den meisten Fällen jedoch biegen sich die serpentinenförmigen Verbindungen im Gleichgewichtszustand außerhalb der Ebene, was bedeutet, dass die Verbindungen bei Biegeverformung nicht in konformem Kontakt mit dem darunter liegenden Substrat stehen. Hochleistungs-Bioelektroden (d. h. Hautsensoren) erfordern einen vollständigen konformen Kontakt (d. h. eine perfekte Schnittstelle) mit der Haut, um die Signalerfassung zu maximieren.

Die Druckschrift US 10,192,830 B2 schlägt zudem nützliche 2D-Designs von selbstähnlichen und fraktalen Designs vor, um dehnbare Verbindungen auf Basis von Serpentinenstrukturen zu entwickeln. Es wurde gezeigt, dass diese fraktalen Verbindungen die maximale Dehnung, die Schaltungen erreichen können, wenn sie in einem Insel-Brücken-Layout angeordnet sind, verbessern. Die Verwendung von fraktalen Designs in der Ebene verbessert wahrscheinlich das Problem der Konformität, das durch geknickte Serpentinenstrukturen außerhalb der Ebene entsteht. Die Verwendung von fraktalen Designs für auxetische Strukturen wird nicht erwähnt. Zudem werden 2D-Vernetzungsmaterialien mit einer null oder positiven Poisson-Zahl demonstriert, die auf der Serpentinenform basieren und in einem fraktalen Muster angeordnet sind. Es wird nicht erwähnt, dass diese Maschenmaterialien ein auxetisches Verhalten aufweisen, außerdem gibt es keine periodischen großflächigen Inseln als Teil der Struktur für die Bauelementeintegration.

Die mechanischen Eigenschaften von auxetischen Materialien machen diese zu idealen Plattformen für dehnbare Elektronik, die in den Körper integriert werden kann. Das Problem bei herkömmlichen auxetischen Strukturen ist, dass die maximale Dehnung, die erreicht werden kann, relativ gering ist und durch die Steifigkeit der Verbindung begrenzt wird. In Grima, Joseph N., et al. "Auxetic behavior from stretching connected squares". Journal of Materials Science 43.17 (2008): 5962-5971 wird das Dehnungsverhalten in rotierenden Quadratmodellen untersucht. Die Quadrate sind an den Ecken durch Scharnierverbindungen miteinander verbunden, die, wenn sie als vollkommen starr angenommen werden, sich einfach relativ zueinander drehen können, um ein auxetisches Verhalten bei angewandter Verformung zu zeigen. Diese Quadrate können auch ein auxetisches Verhalten durch Verlängerung (oder Verkürzung) der Seiten der Quadrate durch den Dehnungsmechanismus erzeugen. In Grima, Joseph N., Elaine Manicaro, and Daphne Attard. "Auxetisches Verhalten von verbundenen Quadraten und Rechtecken unterschiedlicher Größe". Proceedings of the Royal Society A: Mathematical, Physical and Engineering Sciences 467.2126 (2011): 439-458, wird gezeigt, dass rotierende Quadrate und Rechtecke unterschiedlicher Größe miteinander verbunden werden können und trotzdem ein auxetisches Verhalten beibehalten wird.

Einige Beispiele für traditionelle auxetische Strukturen, die modifiziert werden könnten, um eine verbesserte Dehnbarkeit / Kompressibilität zu haben, sind Waben, chirale (Kreis, Quadrat, Sechseck, etc. ), Dreieckstrukturen und Stern, Rautengitter (quadratisch und länglich), Origami-basierte Metamaterialien, Kirigami-basierte Metamaterialien, Key-Bricked-Strukturen, Strukturen mit Schlitzperforation, rotierende starre Strukturen, rotierende halbstarre Strukturen und hierarchische auxetische Systeme.

Die Druckschrift US 8,883,287 B2 offenbart Strukturen, die den auxetischen Strukturen "rotierender Quadrate" ähnlichsehen. In dieser Druckschrift werden die starren Verbindungspunkte zwischen den Quadraten durch Verbindungspunkte mit Zugentlastungsfunktion ersetzt, die die gleiche Form wie eine Serpentinenverbindung haben, ähnlich wie die aus der dehnbaren Elektronik bekannten "Insel-Brückenstrukturen". Die hier vorgestellten Strukturen werden jedoch nicht als auxetisch bezeichnet oder mit einer negativen Poissonzahl belegt. Darüber hinaus müssen die Strukturen sowohl in x- als auch in y-Richtung (biaxial) gedehnt werden und nicht nur uniaxial, um eine Ausdehnung zu erreichen. Mit anderen Worten bedeutet dies, dass die in der Druckschrift vorgestellten Strukturen nicht auxetisch sind. Die aus der klassischen dehnbaren Elektronik bekannten "Insel-Verbindungsstrukturen" können sich auch nur unter biaxialer Dehnung ausdehnen.

Aus dem druckschriftlichen Stand der Technik sind auxetische Gerüststrukturen als flexible Substrate für Funktionskomponenten insbesondere für elektronische Komponenten wie Solarzellen, Antennen, Batterien und Sensoren bekannt. Hierzu wird beispielsweise auf die Druckschriften US 2010/330338 A1, US 2018/061743 A1 und US 2020/144431 A1 verwiesen. Die zuvor genannten Druckschriften offenbaren auxetische Substrate aus klassischen Substratmaterialien wie dem Halbleiter Silizium oder einem Polymer Kunststoff, die selbst keine wesentliche elektrische Leitfähigkeit aufweisen. US 2020/144431 A1 offenbart dabei eine auxetische Steg- oder Feldstruktur aufweisend:
- Inselstrukturflächen und
- Verbindungen zwischen den einzelnen Inselstrukturflächen, wobei
- offene Räume zwischen den einzelnen Inselstrukturflächen vorliegen und die Verbindungen mit den Inselstrukturflächen eine Steg- oder Feldstruktur ausbilden; wobei;
- die Verbindungen die Inselstrukturflächen an derselben Stelle und in demselben Winkel schneiden wie die vorherigen starren Verbinder das auxetische Gerüst;
- die Inselstrukturflächen unter Krafteinwirkung nicht oder nur geringfügig verbiegbar sind,
- wobei die Spannungen auf den Inselstrukturflächen mindestens eine Größenordnung unter denen der dehnbaren Verbindung liegen
   und/oder inderen Größe nicht oder nur unwesentlich veränderlich sind und
- die Poisson-Zahl der Struktur unter uniaxialer Verformung negativ ist.

Ferner ist hinlänglich im Stand der Technik bekannt, Implantate, insbesondere Stents, aus SMA auf Basis von NiTi-Legierungen herzustellen.

Es ist ferner weithin im Stand der Technik bekannt, die Oberflächen solcher medizinischen Implantate mit Beschichtungen zu funktionalisieren.

Herkömmliche auxetische Strukturen können auf verschiedene Weise modifiziert werden, um den Anforderungen eines medizinischen Implantats gerecht zu werden. Großflächige Inseln können periodisch in das Gerüst herkömmlicher auxetischer Geometrien integriert werden, die dem Stand der Technik bekannt sind. Darüber hinaus können die gezackten Scharniere großflächiger auxetischer Geometrien (z. B. rotierende Polygone) durch nachgiebigere Strukturen (z. B. Serpentinen) modifiziert werden, um größere Verformungen zu ermöglichen.

Xue, et al. behandeln in "Design of self-expanding auxetic stents using topology optimization" Frontiers in Bioengineering and Biotechnology 8 (2020): 736 den Zusammenhang zwischen Stentstrukturen und klinischen Ergebnissen bei der Behandlung der Koronararterienerkrankung. Zunächst wird eine Methode zur gleichzeitigen topologischen Optimierung entwickelt, um systematisch die beste Materialverteilung innerhalb des Designbereichs zu finden. Für die Topologieoptimierung wird eine erweiterte parametrische Level-Set-Methode mit Schalenelementen vorgeschlagen, um die Genauigkeit und Effizienz der Berechnungen zu gewährleisten. Anschließend wird ein auxetisches Metamaterial mit negativer Poissonzahl in selbstexpandierenden Stents eingeführt. Die Auxetik kann die mechanischen Eigenschaften der Struktur verbessern, z. B. die Bruchzähigkeit, die Identifikations- und Scherfestigkeit und die Absorption von Vibrationsenergie, was dazu beiträgt, die Nachteile aufgrund mechanischer Ausfälle zu beheben. Abschließend wird der optimierte selbstexpandierende Stent mit der kommerziellen Software ANSYS numerisch validiert und dann mittels additiver Fertigungstechniken als Prototyp hergestellt.

Die Druckschrift US 2017/0007400 A1 zeigt ein Verbund-Biomaterial, das ein durchgehendes Metallblech mit gebogenen Elementen, die ein erstes Fenstermuster definieren, und eine Polymerschicht über mindestens einer Oberfläche des durchgehenden Metallblechs aufweist. Die gebogenen Elemente sind elastisch dehnbar, so dass sich das kontinuierliche Metallblech in mehr als einer Achse biegen kann, ohne zu knicken oder zu falten.

Moderne dehnbare mikroelektronische mechanische Systeme (MEMS) basieren typischerweise auf wellenförmig vermaschten Dünnschicht-Metallen und Inselbrücken-Layouts mit Dünnschicht-Serpentinen-Verbindungen. Dank der Serpentinen- und Wellenstrukturen können extrem große uniaxiale globale Dehnungen erreicht werden. Der große Nachteil der modernen dehnbaren Elektronik ist, dass die dehnbaren Strukturen eine positive oder Null-Poissonzahl haben, was bedeutet, dass sich die Struktur unter einachsiger Zugbelastung entweder zusammenzieht oder in der senkrechten Richtung nicht verändert.

Elektronische Geräte der nächsten Generation, die in biologisches Gewebe integriert werden sollen, müssen minimalinvasiv und ultrakonform sein und die Ko-Integration komplexer mikroelektronischer Schaltungen ermöglichen. Elektronik, die auf verschiedenen Körperoberflächen, z. B. Blutadern, Blutgefäße, Haut, Herzklappen usw. integriert werden soll, muss den extremen Dehnungs-, Biege- und Verdrehungsverformungen standhalten, die mit den natürlichen Krümmungsänderungen des Körpers einhergehen. Beispielsweise sollten elektronische Tattoos, die direkt auf der Haut getragen werden, d. h. Hautsensoren, und medizinische Implantate, die im Inneren des Körpers eingesetzt werden, sich idealerweise an ihre Umgebung anpassen, um die Geräteleistung zu optimieren. Es ist auch wünschenswert, dass die Struktur große spannungsarme Bereiche aufweist, um verschiedene MEMS-Komponenten aufzunehmen und elektrisch zu verbinden. Eine zusätzliche Anforderung an die meisten medizinischen Implantate, z.B. Stents, ist, dass sie auch in der Lage sein müssen, sich um mindestens das ~4-fache ihres Volumens zu komprimieren und auszudehnen, um auf einen ausreichend kleinen Durchmesser eines Mikrokatheters gequetscht zu werden, um durch nichtinvasive endovaskuläre Behandlungen eingesetzt werden zu können.

Der größte Nachteil des Stands der Technik bei auxetischen Strukturen ist, dass die Dehnbarkeit und Komprimierbarkeit der gesamten Struktur durch die Steifigkeit der Verbindungsscharniere (oder Drehgelenke) der Strukturen begrenzt ist. Die Steifigkeit traditioneller auxetischer Strukturen kann ihre praktische Verwendung als implantierbare medizinische Geräte oder andere dehnbare elektronische Anwendungen einschränken, da die Anforderungen an die Volumenexpansion / -kompression mit traditionellen Designs nicht erfüllt werden können.

Im Stand der Technik wurden weiterhin bereits elektronische Komponenten mit SMA-Folien verbunden, und es wurden Stents mit auxetischer Gerüststruktur vorgeschlagen, die naheliegend auch aus einem SMA-Material gebildet werden können.

Problematisch an den im Stand der Technik bekannten Vorrichtungen ist, dass die eine benötigte implantierbare Struktur mit elektronischen Komponenten in der hier im Weiteren beschriebenen Form nicht bekannt ist, jedoch eine derartige Struktur benötigt wird.

Die größte Herausforderung bei der Funktionalisierung herkömmlicher Stentdesigns (das heißt Gitterstrukturen, traditionelle auxetische Strukturen, synthetische Verbundstrukturen) ist die geringe verfügbare Fläche für die Integration elektrischer Geräte. Der nächste Qualitätssprung in der medizinischen Versorgung erfordert eine langfristige, nicht-invasive, unauffällige, kontinuierliche Überwachung der lebenswichtigen biomedizinischen Zeichen. Elektronische Sensoren, die auf verschiedenen Oberflächen des Körpers, bspw. in Blutvenen / Gefäßen / Arterien, in den Herzklappen, auf der Haut, auf den Knochen usw. integriert werden können, müssen sich an diese komplexen Oberflächen anpassen und den extremen Dehnungs-, Biege- und Verdrehungsverformungen standhalten, die mit den natürlichen Krümmungsänderungen des Körpers einhergehen. Das Hauptproblem bei herkömmlichen elektronischen Komponenten ist, dass sie starr sind und sich typischerweise nicht an gekrümmte Oberflächen anpassen können.

Die nächste Generation medizinischer Implantate wird funktionalisiert sein, um Patientendaten in Echtzeit drahtlos an die Ärzte zu übertragen und so eine bessere Behandlungsqualität zu ermöglichen. Herkömmliche Stents, die auf gewebten, geflochtenen und lasergeschnittenen Stents aus Formgedächtnislegierungen basieren sind etwas starr und können sich nicht vollständig an die Form der Vene anpassen. Darüber hinaus bieten herkömmliche Stents keinen so hohen Flächenanteil an spannungsfreien Bereichen, wie ihn aktive oder passive Funktionskomponenten aufnehmen könnten. Diese Eigenschaft ist für zukünftige miniaturisierte funktionalisierte medizinische Implantate notwendig, um die Integration für drahtlose Übertragungsantennen, Energy Harvesting Devices, Energiespeicher und andere Arten von Sensor- oder Aktorkomponenten zu ermöglichen.

Die Druckschrift US 10,660,200 B2 offenbart, dass archimedische Spiraldesigns wesentlich dehnbarer sind als Serpentinen- und Fraktaldesigns, die derzeit für dehnbare Elektronik verwendet werden. Archimedische Spiralen weisen deutlich geringere Spannungen auf als Serpentinenverbindungen. Sie können so große einachsige Verschiebungen durch Knicken und Verdrehen außerhalb der Ebene erreichen. Die in der Druckschrift offengelegten Strukturen verbinden die Spiralen durch ein bekanntes Insel-Brücken-Layout, bei dem die archimedische Spirale die Insel direkt in der Mitte der Fläche miteinander verbindet. Der Verbindungspunkt in der Mitte der Fläche der Inseln ist der Grund dafür, dass die Struktur insgesamt eine positive oder null Poissonzahl aufweist.

Das oben beschriebene Problem der geringeren Dehnbarkeit von auxetischen Strukturen kann gelöst werden, indem das starre Scharnier- oder Drehgelenk einer auxetischen Struktur durch dehnbare Verbindungen modifiziert wird. Ein erstes Beispiel dafür ist die Druckschrift US 2020/0144431 A1, in der gezeigt wird, dass die mechanischen Eigenschaften von auxetischen Strukturen durch die Verwendung von Serpentinen-Verbindungen verbessert werden können. Die Druckschrift offenbart eine verformbare Anordnung von Halbleiterbauelementen und ein Verfahren zur Herstellung einer solchen verformbaren Anordnung. Das verformbare Array umfasst eine Vielzahl von Inseln, wobei jede Insel mindestens ein Halbleiterbauelement enthält und die Vielzahl von Inseln in einer auxetischen Geometrie angeordnet sind. Die Verbindungen der Inseln miteinander sind, wie bereits genannt, als Serpentinen ausgebildet und jede Serpentine enthält auch ein Halbleiterbauelement. Die hier offenbarte Struktur ist in der Lage sich unter einachsiger Belastung auszudehnen.

Aufgabe der vorliegenden Erfindung ist es, die vorteilhaften mechanischen Eigenschaften von auxetischen Strukturen und dehnbaren Zwischenverbindungen so zu kombinieren, dass eine auxetische Struktur geschaffen wird, die besonders dehnbar ist und gleichzeitig ausreichend große Flächen bereitgestellt werden, die allenfalls nur geringen Spannungen ausgesetzt sind.

Die vorliegende Erfindung löst die oben genannten technischen Probleme, indem sie eine mechanisch dehnbare auxetische Struktur vorstellt. Diese Struktur nutzt die überlegenen mechanischen Eigenschaften, die auxetische metamechanische Materialien (im Folgenden als auxetisch bezeichnet) bieten, und verbessert ihre Dehnbarkeit und Komprimierbarkeit durch die Integration von Zwischenverbindungen in das auxetische Gerüst.

Auxetische Materialien sind bekannte periodische Strukturen, die durch eine negative Poissonzahl gekennzeichnet sind, das heißt sie dehnen sich seitlich aus, wenn sie in Längsrichtung gedehnt werden. Unter einer einachsigen Zugkraft dehnen sich diese Strukturen in der senkrechten Richtung der aufgebrachten Kraft aus oder ziehen sich zusammen. Die Strukturen weisen beeindruckende Materialeigenschaften auf, darunter synklastische Biegung, hohes Steifigkeits-Gewichts-Verhältnis, große Scherfestigkeit, große Härtewerte, hohe Eindrück-/Stoßfestigkeit, geringere Ausbreitung von Ermüdungsrissen, größere Zähigkeit und Modulbeständigkeit sowie Schwingungsdämpfung. Insbesondere die synklastische Biegung bietet einzigartige Möglichkeiten für Anwendungen in der tragbaren Elektronik und in der Medizintechnik, da sie konformes Biegen um konkave, konvexe sowie konvexes Biegen um konkave, konvexe, kuppelförmige Kurven und andere komplexe, nichtlineare 3D-Geometrien ermöglicht, wodurch sich zweidimensionale auxetische Strukturen an jede dreidimensionale Oberfläche anpassen können.

Es sollen insbesondere die Expansions- / Kompressionsanforderungen durch die hier vorgestellte auxetische Struktur erfüllt werden, die beispielsweise von funktionalisierten medizinischen Implantaten benötigt werden. Somit soll ein flexibles, sehr oft verbiegbares Substrat für elektronische Komponenten aufgezeigt werden, dass die Eigenschaft hat, biokompatibel und implantierbar zu sein, so dass Körperbewegungen zulässig sind und das Substrat und dessen Struktur sowie die darauf befindlichen elektronischen Komponenten nicht durch Bewegungen zerstört oder beschädigt werden.

Eine weitere Aufgabe kann darin ergänzend gesehen werden, die Kombination von Formgedächtnislegierungen als Rückgrat der auxetischen Struktur für ein neues Design für medizinische Geräte zu realisieren bzw. zu nutzen, die durch nicht-invasive endovaskuläre Behandlungen in Herz und Gehirn eingesetzt werden können.

Gelöst werden diese Aufgaben mit einer auxetischen Steg oder Feldstruktur gemäß dem Hauptanspruch 1.

Die auxetische Steg- oder Feldstruktur weist Inselstrukturflächen und Verbindungen zwischen den einzelnen Inselstrukturflächen auf, wobei offene Räume zwischen den einzelnen Inselstrukturflächen vorliegen und die Verbindungen mit den Inselstrukturflächen eine Steg- oder Feldstruktur ausbilden, dadurch gekennzeichnet, dass die Verbindungen dehnbar sind und als archimedische Spiralverbindungen und / oder selbstähnliche fraktale Designverbindungen ausgestaltet sind, die Verbindungen die Inselstrukturflächen an derselben Stelle und in demselben Winkel schneiden, wie die vorherigen starren Verbinder das auxetische Gerüst, die Inselstrukturflächen unter Krafteinwirkung nicht oder nur geringfügig verbiegbar sind, wobei die Spannungen auf den Inselstrukturflächen mindestens eine Größenordnung unter denen der dehnbaren Verbindung liegen, und / oder in deren Größe nicht oder nur unwesentlich veränderlich sind und die Poisson-Zahl der Struktur unter uniaxialer Verformung negativ ist.

Es werden somit dehnbare Bauelemente durch die Kombination von auxetischen Geometrien aus der Literatur mit dehnbaren Verbindungen bereitgestellt. Die auxetischen Strukturen zeichnen sich durch eine negative Poissonzahl aus, was bedeutet, dass sich die Struktur unter einachsiger Zugbelastung in der senkrechten Richtung der aufgebrachten Kraft ausdehnt.

In ähnlicher Weise ziehen sich die hier vorgestellten Strukturen bei einachsiger Druckbelastung aufgrund der negativen Poissonzahl auch in der senkrechten Richtung zusammen.

Jedes Dünnschichtmaterial, das in die in dieser Erfindung offenbarten Strukturen eingearbeitet wird, zeigt auxetische Eigenschaften mit verbesserter Dehnbarkeit und Kompressibilität.

Der erfindungsgemäße Gegenstand ermöglicht eine noch höhere Dehnbarkeit als in der Druckschrift US 2020/0144431 A1 offenbart, da die in der genannten Offenbarung beschriebenen Serpentinenverbindungen durch Verbindungen ersetzt werden, die bekanntermaßen eine höhere Dehnbarkeit aufweisen, nämlich selbstähnliche und /oder fraktale Designverbindungen und / oder archimedische Spiralverbindungen. Theoretisch könnten die mechanischen Eigenschaften vieler anderer auxetischer Strukturen verbessert werden, indem ihre starren Verbindungen durch dehnbare ersetzt werden.

Die archimedische Spiralstruktur hat die größte elastische Dehnbarkeit von bis zu 200 %, während die reguläre Serpentine und die selbstähnliche Serpentine eine elastische Dehnbarkeit von 112 % bzw. 98 % aufweisen. Daher können dehnbare auxetische Strukturen mit archimedischen Verbindungen im Vergleich zu auxetischen Strukturen mit dehnbaren Serpentinenverbindungen, fraktalen Verbindungen und herkömmlichen starren Scharnieren eine enorme Dehnbarkeit und größere Volumen- und Flächenausdehnungen erreichen. Die gewünschten Eigenschaften (wie Dehnbarkeit, etc.) der erfindungsgemäßen auxetischen Steg- oder Feldstruktur werden zudem dadurch, dass die Verbindungen die Inselstrukturflächen an derselben Stelle und in demselben Winkel schneiden wie die vorherigen starren Verbinder das auxetische Gerüst weiter verbessert.

Die vorliegende Erfindung erreicht im Vergleich zu herkömmlichen auxetischen Strukturen eine größere radiale Ausdehnung und eine kleinere radiale Kompression. Dies wird erreicht, indem die starren Verbindungspunkte der Inselstrukturflächen durch flexible / dehnbare archimedische Spiralverbindungen oder selbstähnliche fraktale Designverbindungen ersetzt werden. Dehnbare auxetische Strukturen könnten durch unterschiedlich große Inselstrukturflächen in Kombination mit unterschiedlich großen/ geformten dehnbaren Zwischenverbindungen gebildet werden.

Jedes Material, das in diese Art von dehnbarer auxetischer Struktur verarbeitet wird, kann von den verbesserten Materialeigenschaften profitieren. Aufgrund der negativen Poissonzahl können sich auxetische Strukturen an nahezu jede konkave oder konvexe Oberfläche anpassen. Diese Eigenschaft führt zu einer synklastischen Biegung, die es auxetischen Strukturen ermöglicht, sich an nichtlineare Oberflächen anzupassen. Durch das Ersetzen der traditionellen "Drehgelenke" oder starren Verbindungspunkte traditioneller auxetischer Strukturen durch gewundene Stegverbindungen, nämlich insbesondere selbstähnlich fraktale Designverbindungen und / oder archimedische Spiralen, wird die Dehnbarkeit und Kompressibilität der auxetischen Struktur um mehrere Größenordnungen verbessert.

Die Verbindungen können hierbei zusätzlich zumindest abschnittsweise folgende Eigenschaften aufweisen: bogenförmig und / oder knickfrei und / oder mit Biegeradien und / oder bifilar und / oder schraubenförmig.

Archimedisch inspirierte Designs für dehnbare Verbindungen sind sehr vorteilhaft, da bereits gezeigt wurde, dass sie eine Dehnbarkeit von mehr als 1020 % erreichen können, wenn die Verbindung mit einem hohen Amplituden- / Breitenverhältnis entworfen wird. Es wird angenommen, dass eine gleichmäßige und kleine Krümmung zu einer größeren Dehnbarkeit solcher Strukturen beiträgt. Zudem wären auch komplexe Spiralformen denkbar, die zumindest Teile von bekannten Spiralen umfassen.

Insbesondere können die einzelnen Inselstrukturflächen und / oder die einzelnen Verbindungen der auxetische Steg- oder Feldstruktur in Größe und Gestalt variierend ausgebildet sein. So wird ein an die Erfordernisse angepasstes Gesamtdesign der auxetischen Struktur möglich.

Die auxetische Steg- oder Feldstruktur kann zusätzlich wenigstens abschnittsweise über Bereiche gleich und / oder periodisch ausgebildet sein.

Die Inselstrukturflächen können zusätzlich auch gleichartig ausgebildet sein. Die Inselstrukturflächen können eine große Fläche einnehmen, wobei durch die Verbindungen eine spannungsfreie Ausdehnung weiterhin möglich ist. Die Inselstrukturflächen können auf die richtige Größe gebracht werden, um jede Art von herkömmlichen starren elektronischen MEMS-Komponenten aufzunehmen. Die dehnbare auxetische Struktur kann so konstruiert werden, dass ein Kompromiss zwischen der Dichte der "Einheitszellfläche" und der maximalen Volumenausdehnung der gesamten Struktur besteht.

Insbesondere können die selbstähnlichen fraktalen Designverbindungen Koch'sche Linien, Peano'sche Linien, Hilbert'sche Linien, Moore'sche Schleifen, Vicsek'sche Schleifen und verzweigte Netze wie das griechische Kreuz umfassen.

Die auxetische Steg- oder Feldstruktur kann planar, 2D hergestellt werden und nachfolgend an 3D-Oberflächen angepasst werden, wobei ein 3D-auxetisches Verhalten beibehalten wird. Dies ist insbesondere möglich, wenn die 2D hergestellte Struktur aus einer amorphen Formgedächtnislegierung gebildet ist.

Die Freiräume zwischen den Inselstrukturflächen und den Verbindungen können maschenartig ausgebildet sein und potenziell als Raum für eine weitere Funktionalisierung des Geräts oder einfach nur als dehnbare Verbindung dienen.

Vorzugsweise kann die auxetische Steg- oder Feldstruktur wenigstens einzelne Inselstrukturflächen zur Aufnahme von Elektronik oder einzelne mit Elektronik bestückte Inselstrukturflächen aufweisen.

Die auf den Inselstrukturflächen befindliche Elektronik kann über zumindest eine von der auxetischen Steg- oder Feldstruktur elektrisch isolierten Leiterbahn auf wenigstens einer Verbindung der auxetischen Steg- oder Feldstruktur miteinander elektrisch verbunden sein.

Insbesondere kann die erfindungsgemäße auxetische Steg- oder Feldstruktur als implantierbare Struktur aus einem biokompatiblen Material, wobei das biokompatible Material eine metallische Formgedächtnislegierung (SMA) ist und die auxetische Steg- oder Feldstruktur metallisch ausgebildet ist., verwendet werden.

Hierbei kann die implantierbare Struktur eine selbstexpandierende Implantatform mit einer Zylinder- und / oder Kugel- und / oder Halbkugel- und / oder Röhren- und / oder gekrümmten Röhrenstruktur wenigstens abschnittsweise aufweisen.

Mit der erfindungsgemäßen auxetischen Steg- und Feldstruktur wird also ein extrem verformbares metallisches auxetisches Substrat mit großen Flächen bereitgestellt, das für die Integration von funktionalen elektronischen Geräten geeignet ist.

Die elektronischen Geräte können dabei auch spröde sein, z. B. Keramiken umfassen, und bleiben bei Gerüstbiegungen gleichwohl intakt und an den Inselstrukturflächen haften.

Elektrisch gut leitende Verbindungen aus Metall zwischen verschiedenen Inseln bzw. Inselstrukturflächen einer Gerüststruktur müssen präzise auf die Verbindungen zwischen den Inselstrukturflächen aufgebracht werden, was mit kleineren Strukturabmessungen immer kostspieliger und aufwendiger wird. In der Regel sind elektronische Komponenten oder Bauelemente wenigstens Zweipole, die elektrische Spannungen erzeugen bzw. auf Spannungssignale reagieren. Es ist daher wünschenswert und bei Belegung eines Großteils der Inselstrukturflächen mit Bauelementen praktisch unvermeidlich, dass jede Verbindung einer Gerüststruktur mindestens zwei metallische, aber voneinander elektrisch isolierte Pfade zur Signalleitung trägt. Die Isolierung der beiden Pfade darf dabei nicht zu dünn sein, da auch geringe Spannung sonst zu lokal hohen Feldstarken und damit elektrischen Durchbrüchen führen können. Speziell bei Gerüststrukturen, die zur Implantation in Gefäßen eines Patienten vorgesehen sind, müssen die Strukturabmessungen klein und die Breiten der Verbindungen / Stege erst recht im Bereich weniger zehn Mikrometer ausgelegt sein. Wenn schmale Stege von mehr als einer metallischen Leiterbahn belegt sind, sind die Spannungssignale, die über das Netz übertragen werden können, stark eingeschränkt. Dies kann aus Sicherheitsgründen zu Einschränkungen bei der Auswahl der elektronischen Komponenten und/oder bei der Gestaltung der Netzarchitektur führen.

Durch die Herstellung von Schaltkreisen auf auxetisch strukturierten

Formgedächtnislegierungssubstraten wird eine extreme Flexibilität innerhalb des gesamten elektronischen Systems ermöglicht. Die vorteilhaften Materialeigenschaften der Formgedächtnislegierung (SMA) gepaart mit den überlegenen mechanischen Eigenschaften der auxetischen Strukturen ermöglichen es den Substraten und allen darauf gefertigten Komponenten, sich an die verschiedenen Oberflächen des Körpers anzupassen. Medizinische Implantate, die z. B. in einem Hirngefäß eingesetzt werden sollen, sollen ein Leben lang halten, daher müssen alle Materialien und Gerätekomponenten so konstruiert sein, dass sie Millionen von Belastungszyklen standhalten. Die SMAs NiTi sind biokompatibel, d. h. sie bauen sich im Körper des Patienten nicht ab und geben keine schädlichen Stoffe ab. Außerdem können einige SMA superelastisch und ohne Ermüdung verformt werden.

Aus dem Stand der Technik ist nur aus der Druckschrift US 2020/0144431 A1 ein metamechanisches auxetisches Design mit Inselstrukturflächen und Verbindungen, entsprechend den hier beanspruchten kleinen miteinander elektrisch verbundenen Inselstrukturflächen für die funktionelle Geräteintegration vorhanden. Im Unterschied zu der genannten Druckschrift weist die erfindungsgemäße auxetische Steg- oder Feldstruktur archimedische Spiralverbindungen und / oder selbstähnliche fraktale Designverbindungen auf. In der Druckschrift werden ausschließlich Serpentinenverbindungen genutzt. In der erfindungsgemäßen auxetischen Steg- oder Feldstruktur schneiden die Verbindungen die Inselstrukturflächen an derselben Stelle und in demselben Winkel, wie die vorherigen starren Verbinder das auxetische Gerüst. Zu dem Schnittwinkel finden sich keine Angaben in der genannten Druckschrift. Somit scheint dieser dort allenfalls nur eine untergeordnete oder eigentlich gar keine Bedeutung zu haben. Gleichwohl ist der Schnittwinkel von zentraler Bedeutung für die erfindungsgemäße auxetische Steg- oder Feldstruktur.

Mit der erfindungsgemäßen auxetischen Steg- oder Feldstruktur wird das Vorurteil, dass freitragende Metallverbindungen mechanisch nicht robust genug sind, um freistehende Stegstrukturen zu sein, durch die Verwendung von Formgedächtnislegierung überwunden. Es kann zudem ein Array aus verschiedenen funktionalen Bauelementen hergestellt werden, dass über eine Vielzahl von Inselstrukturflächen verteilt angeordnet ist, die wiederum miteinander elektrisch über die Stege verbunden sind.

Jede einzigartig modifizierte auxetische Geometrie bietet einen Kompromiss aus mechanischer Flexibilität, Dehnbarkeit, Verformbarkeit und Flächendichte und bietet insgesamt eine Fülle möglicher anpassungsfähiger Formen, die für das Design medizinischer Geräte der nächsten Generation verwendet werden könnten.

Insbesondere kann die Formgedächtnislegierung aus NiTi sein. SMAs werden hierbei als auxetische Backbones für medizinische Implantate verwendet, da einige biokompatible SMAs auf NiTi-Basis bereits in Versuchen gezeigt haben, dass sie die Zyklenanforderungen erfüllen können. Das SMA kann auch eine der NiTi-Legierungen wie TiNiCu, TiNiCuCo, TiNiHf und ähnliche sein.

Weiter können die Elektronikkomponenten aus verschiedenen Elementen ausgewählt sein, wie Piezoelement, Energiespeicher, Antennenstruktur, vorgefertigte Mikroprozessoren. Beispiele für aktive Elektronik, die auf den Inselstrukturflächen und / oder dem Rückgrat der auxetischen Struktur integriert werden könnten, sind unter anderem: Elektroden, Sensoren (chemisch, biologisch, elektrisch, magnetisch, mechanisch), Aktoren, Verbindungen, Kondensatoren, LEDs, Batterien und Solarzellen zur Energiespeicherung, Energy Harvester und Antennen. Diese Komponenten können im Wesentlichen aus jedem Material geformt werden, das derzeit mit modernen Mikrobearbeitungs- und Abscheidetechniken kompatibel ist. Beispiele für Materialien sind unter anderem Metalle, Oxide, Dielektrika, Perowskite, Halbleiter, Polymere, Keramiken, piezoelektrische, pyroelektrische, magnetische, ferroelektrische und magnetostriktive Materialien.

Zusätzlich könnten auxetische Strukturen aus Formgedächtnislegierungen mit anderen Arten von Aktuatorvorrichtungen aus Formgedächtnislegierungen funktionalisiert werden.

Zusätzlich zu auxetischen SMAs, die ein patientenspezifisches Design für medizinische Implantate bieten, bietet die Funktionalisierung eine neuartige Eigenschaft, indem sie die drahtlose Übertragung von vitalen Patientendaten nach außen ermöglicht.

Auxetische Formgedächtnislegierungen, die mit einem anderen Formgedächtnis mit einer anderen Übergangstemperatur gekoppelt sind, könnten verwendet werden, um selbstfaltende auxetische Strukturen zu schaffen.

Ein weiteres Merkmal ist, dass die hier zur Anwendung kommenden Formgedächtnislegierungen elektrisch leitfähige Materialien sind, deren Leitfähigkeit nur ~1 Größenordnung schlechter ist als die konventioneller Metalle, wie Platin, Kupfer und Gold. Die elektrische Leitfähigkeit von SMAs ähnelt der von Flüssigmetallen, die als eines der dehnbarsten Metalle gelten. Die größte Herausforderung bei Flüssigmetallen und konventionellen Dünnschichtmetallen besteht darin, dass sie in ein elastomeres Substrat integriert werden müssen. Die relativ hohe Leitfähigkeit der Formgedächtnislegierung gepaart mit ihrer mechanischen Robustheit ermöglicht es, dass das freistehende Substrat als Masseelektrode zur Verbindung aller aktiven elektronischen Komponenten dient. Die Bauteile können auf einzelnen Inselstrukturflächen gefertigt und dann durch ein metallisches SMA-Substrat elektrisch verbunden werden. Die kombinierte Festigkeit des SMA-Materials und der auxetischen geometrischen Struktur führt zu geringen Spannungen unter angewandter Belastung in der gesamten auxetischen Struktur. Daher könnte die gesamte auxetische Rückgratstruktur so konstruiert werden, dass sie andere Dünnfilm-Materialspuren und sogar potenziell vollständige Geräte aufnehmen kann.

Es ist somit auch möglich die auxetische Steg- oder Feldstruktur zur Verbesserung des Stands der Technik als gemeinsame Masseelektrode ausgebildet vorzusehen.

Alternativ oder auch ergänzend können wenigstens zwei Leiterbahnen nebeneinander beabstandet und / oder übereinander jeweils mit einer Isolationsschicht zwischen der metallischen auxetischen Struktur und der Leiterbahn vorgesehen sein.

Der unmittelbare Vorteil liegt darin, dass ein zweiter metallischer Pfad über einen Steg in recht beliebiger Weise von der Masseelektrode isoliert und insbesondere beabstandet werden kann, beispielsweise durch eine vergleichsweise dickere Dielektrikumsschicht zwischen beiden. Anders gesagt, man kann über die einzelnen Verbindungen / Stege der Gerüststruktur jeweils eine Mehrzahl von gut leitenden Pfaden für Spannungssignale vorsehen, wobei mindestens ein Pfad existiert, der eine vorgebbare Maximalspannung ohne Schädigung des Implantats verkraften kann. Dies erweitert die Möglichkeiten der Planung einer solchen Gerüststruktur und ihrer elektronischen Bestockung, letztlich die endgültige Funktionalität eines flexiblen elektronischen Implantats.

In einer Ausgestaltung kann wenigstens oberhalb der elektrischen Verbindung eine abdeckende Isolationsschicht und / oder weitere Isolationsschichten vorgesehen sein.

Weiter kann die Isolationsschicht und / oder weitere Isolationsschichten von isolierten Leiterbahnen bevorzugt aus einem nicht-leitenden Oxid bestehen. Ein derartiges Material kann auch mittels der in dieser Offenbarung ausgeführten Verfahren aufgebracht werden.

Ferner kann ebenfalls in einer bevorzugten Variante die implantierbare Struktur wenigstens abschnittsweise als äußere Schicht ein biokompatibles Polymer aufweisen.

Besonders bevorzugt ist auf den wenigstens auf der auxetischen metallischen SMA-Struktur vorgesehenen Schichten und Komponenten, eine insbesondere biokompatible Schicht aufgebracht. Eine auxetische metallische SMA-Struktur kann in einer Ausführungsvariante hierbei frei von einer Überdeckung bleiben, da hierfür biokompatibles Material verwendet wird.

Bevorzugt kann das Struktur-Aspektverhältnis, also Strukturbreite zur Strukturdicke, der Leiterbahn wenigstens im Bereich der Stege oder der Stegstruktur im Bereich von 0,5 bis 1 liegen.

Weiter kann die Strukturdicke der Leiterbahn im Bereich von ~10 nm bis zu 200 µm liegen. Für medizinische Implantate dürfte die Zieldicke der Gesamtstruktur durchaus in den Bereich von ~30-50µm gehen. Für ein tragbares E-Tattoo wären jedoch Leiterbahnen bis zu 200 µm möglich, da keine räumliche Anforderung an die Bauteilgröße besteht.

Ein mögliches Herstellungsverfahren einer implantierbaren Struktur aus einem biokompatiblen Material weist die folgenden Schritte auf:
- Herstellen einer auxetischen Steg- oder Feldstruktur mit Inselstrukturflächen und Verbindungen, wobei offene Räume zwischen den einzelnen Inselstrukturflächen vorliegen und die Verbindungen mit den Inselstrukturflächen eine Steg- oder Feldstruktur ausbilden, wobei wenigstens einzelne Inselstrukturflächen zur Aufnahme von Elektronik oder einzelne mit Elektronik bestückte Inselstrukturflächen ausgebildet werden und wenigstens diese Inselstrukturflächen unter Krafteinfluss auf die auxetische Steg- oder Feldstruktur nicht oder nicht wesentlich verbiegbar und/oder in deren Größe nicht oder nur unwesentlich veränderlich sind, wobei hierzu das biokompatible Material aus einer metallischen Formgedächtnislegierung gewählt wird und die auxetische Struktur aus einer metallischen auxetischen Struktur gebildet wird,
- Herstellen einer auf wenigstens zwei Inselstrukturflächen verteilten und miteinander elektrisch verbundenen Elektronik und / oder Elektronikkomponenten und einer elektrischen Verbindung zwischen der Elektronik und / oder den Elektronikkomponenten, wobei diese elektrische Verbindung durch Herstellen wenigstens einer von der metallischen auxetischen Struktur elektrisch isolierten Leiterbahn auf wenigstens einer Stegstruktur der metallischen auxetischen Struktur erfolgt.

Die wenigstens eine Leiterbahn und / oder die wenigstens eine Isolierschicht und / oder die Elektronik und / oder die Elektronikkomponenten auf der auxetischen Struktur können besonders bevorzugt aufgesputtert und / oder auch bevorzugt mit einer weiteren Dünnschichttechnik, Sol-Gel-Technik oder einem chemischen oder physikalischen-Depositions-Verfahren aufgebracht werden.

Weiter kann die wenigstens eine Leiterbahn und / oder die wenigstens eine Isolierschicht zusammen mit der Elektronik und / oder den Elektronikkomponenten auf der auxetischen Struktur aufgebracht werden. Es ist durchaus möglich, entsprechende Schritte gemeinsam zu vollziehen und so synergetische Vorteile bei der Konstruktion und Herstellung zu erzielen.

Durch die erfindungsgemäße Modifikation traditioneller auxetischer Designs, wird die Integration spannungsarmer Inselstrukturflächen in das Grundgerüst der auxetischen Strukturen möglich. Diese Inselstrukturflächen werden bei mechanischer Belastung, z. B. durch Crimpen oder Dehnen, kleinen Spannungen ausgesetzt. Die Inselstrukturflächen können auf die richtige Größe gebracht werden, um jede Art von traditionellen starren elektronischen MEMS- oder auch NEMS-Komponenten aufzunehmen.

Die Verwendung von Formgedächtnislegierungen bietet einen großen Vorteil in der Merkmalskombination, da sich die auxetische Struktur durch eine spannungs- oder temperaturinduzierte Fest-zu-Fest-Phasenumwandlung auf extrem große Volumina selbst ausdehnen kann.

Das Ausmaß der radialen Volumenexpansion wird weiter erhöht, indem die starren Verbindungspunkte der Inselstrukturflächen durch dehnbare Zwischenverbindungen, d. h. archimedische Spiralverbindungen und / oder selbstähnliche fraktale Designverbindungen ersetzt werden.

Die Implementierung von Formgedächtnislegierungen als Rückgrat der auxetischen Struktur bietet bessere mechanische Eigenschaften für das Gerät / Device, d.h. eine längere Lebensdauer des Geräts bzw. des Devices. Der Formgedächtniseffekt ist der Schlüssel dazu, dass es für das modifizierte auxetische Substrat möglich wird eine hohe Eigenspannung aufzunehmen, so dass das medizinische Implantat verformungsbeständig ist. Das Hinzufügen von funktionalisierten Inselstrukturflächen erhöht die Fähigkeiten des Geräts, indem Elemente wie Drucksensoren und Dehnungssensoren und weitere hinzugefügt werden. Die Entzündungsreaktion des Körpers auf die Implantation des Geräts könnte auch durch direkte lokale Medikamentenfreisetzung in einer Ausführungsvariante reduziert werden. Ein solches medizinisches Implantat ermöglicht somit die direkte lokale Verabreichung von Medikamenten an die vorgesehene Stelle im Körper, was im Allgemeinen die Nebenwirkungen von Medikamenten, die auf herkömmliche Weise (z. B. oral) verabreicht werden, verringert.

Grundsätzlich können SMA gekrimpt, d. h. auf ein kleines Volumen zusammengefaltet werden (ohne dabei die Inselstrukturflächen zu biegen) und entfalten sich selbst bei Körpertemperatur im Innern des Patienten. Dies erlaubt u.a. ein minimal-invasives Positionieren im endoskopischen Eingriff.

Ein bevorzugter Prozess zur Herstellung basiert auf der Herstellung aller Gerätekomponenten mit MEMS-Verarbeitungstechniken [micro-/nanoelectromechanical systems (MEMS/NEMS)] auf einem amorphen auxetischen SMA-Substrat unter Verwendung eines in der US 8,758,636 B2 (auch als Lima et al. bezeichnet) veröffentlichten Prozesses. Der gesamte Verbundwerkstoff wird dann durch Ausglühen (> 450 °C) in eine komplexe 3D-Form gebracht, um das SMA zu kristallisieren. Die nützlichen Formen, die hier vorgesehen sind, sind ein zylindrisches 3D-Rohr für Stents und eine 3D-Kugel oder Halbkugel für intrasakkuläre Aneurysma-Geräte, die auf ausreichend kleine Abmessungen gequetscht werden können, um das Gerät durch einen Mikrokatheter einzuführen. Diese Eigenschaft würde es ermöglichen, dass die Geräte durch endovaskuläre Behandlungen eingesetzt werden können, die nicht-invasiv und sicherer für den Patienten sind, d.h. kürzere Operationszeit, geringere Belastung durch Röntgen- und MRT-Strahlung. Das Verfahren kann angewendet werden, um amorphe Formgedächtnislegierungsmaterialien in die gewünschten 2D-auxetischen Strukturen zu strukturieren. Dieser Prozess ermöglicht eine hohe geometrische Präzision mit Feature-Größen von bis zu 10 Mikrometern unter Verwendung von Photolithographie-, Sputtering- und chemischen Wet-etch-Release-Prozessen. Da die SMA amorph mit einer geringen Oberflächenrauhigkeit abgeschieden werden können, können sie bis zu jeder Temperatur (< 800 °C) weiterverarbeitet werden, was das SMA-Substrat mit den meisten modernen MEMS-Prozessen kompatibel macht. Modernste Substrate, die derzeit in Implantaten und in dehnbaren Bauteilen verwendet werden, wie Polymere und Elastomere, können diese hohen Temperaturen (< 200 °C) nicht verarbeiten.

Während bisher bekannte kommerzielle Stents sich im Körper zusammenziehen und entfalten können, enthalten die meisten Designs ein etwas starres Rückgrat, das sich nicht an die Form der Arterie anpassen kann. Auxetische Stentstrukturen würden bekannten Stents überlegen sein, da sie patientenspezifisch werden und sich an jede Art von komplexer geometrischer Oberfläche anpassen können, was notwendig ist, da die Hirngefäße, Arterien und Aneurysmen jedes Patienten unterschiedliche Formen und Größen haben. Nach dem Stand der Technik weisen auxetische Stentstrukturen höhere Radialkräfte am Stentumfang auf, sogar 10-mal höher als bei aktuellen Stentdesigns. Darüber hinaus bieten die aktuellen Stentdesigns keine großen Inselstrukturflächen, die mit aktiven und passiven Geräten integriert oder gar funktionalisiert werden könnten.

Wie bereits zuvor dargestellt, kann die hier beschriebene Erfindung als Plattform für die Funktionalisierung jeder Art von elektronischem Gerät auf praktisch jeder Art von komplexer geometrischer Oberfläche (von der Makro- bis zur Nanoskala) unter Verwendung von insbesondere auxetischen Formgedächtnislegierungssubstraten dienen.

Jede einzigartig modifizierte auxetische Geometrie bietet einen Kompromiss aus mechanischer Flexibilität, Dehnbarkeit, Verformbarkeit und Flächendichte und bietet insgesamt eine Fülle möglicher anpassungsfähiger Formen, die für das Design der nächsten Generation dehnbarer Elektronik nützlich sein könnten. Die meisten dehnbaren Anwendungen haben ähnliche Ziele: Maximierung der Dehnbarkeit und Maximierung der Flächenabdeckung. d.h. mehr Funktionsflächen für die Bauteilintegration zu ermöglichen.

Ein großer Vorteil des neuartigen dehnbaren auxetischen Designs ist, dass alle Designs unter angelegter Zug-/Druckspannung große Inselstrukturflächen mit geringer Spannung bieten, die geeignet sind, im Wesentlichen jede Art von MEMS-Komponente aufzunehmen. Diese Schlüsseleigenschaft kann theoretisch die Ko-Integration verschiedener elektronischer Komponenten, wie z.B. Batterien, Antennen, Sensoren, Aktoren, Solarzellen, und dgl. durch das Substrat oder eine leitfähigere Elektrode, die auf dem dehnbaren auxetischen Substrat aufgebracht ist, ermöglichen. Halbleiterbauelemente könnten direkt in die selbstähnliche und / oder fraktale und / oder archimedische Verbindungsform gemustert werden, um verformbare Halbleiterarrays mit ähnlichen Methoden zu schaffen, wie in der Druckschrift
US 2020/0144431 A1 beschrieben.

Die großen Inselstrukturflächen in den hier aufgezeigten Designs unterliegen geringen Spannungen unter angewandten Dehnungen, die elektronische Komponenten für piezoelektrische Energy Harvester und Halbleitergeräte wie Displays und Solarzellen und für medizinische Implantate aufnehmen können.

In einer bevorzugten Ausführungsform kann die Struktur darüber hinaus in ihrer Gesamtheit oder in Abschnitten ein selbstähnliches fraktales Design aufweisen, um die Kompressibilität und Dehnbarkeit der auxetischen Struktur zu verbessern. In einer anderen Ausführungsform kann die angeordnete (oder stegförmige) auxetische Struktur in ihrer Gesamtheit oder in Abschnitten archimedische Spiralverbindungen aufweisen.

Die Poissonzahl der gesamten modifizierten auxetischen Struktur ist insbesondere negativ, was bedeutet, dass sich die Struktur unter einachsiger Zugbelastung in der senkrechten Richtung ausdehnt. Die negative Poissonzahl von auxetischen Strukturen ist für dehnbare Elektronik vorteilhaft, da sich die Strukturen unabhängig von Material und Dicke an jede kurvige Oberfläche anpassen können. Moderne dehnbare mikroelektronische mechanische Systeme (MEMS) basieren in der Regel auf wellenförmig vernetzten Dünnfilm-Metallen und Inselbrücken-Layouts mit Dünnfilm-Serpentinen-Verbindungen. Darüber hinaus soll die dehnbare Elektronik beim Dehnen zeitgleich eine große Flächendichte für die Integration der Bauelemente beibehalten.

Zu den weiteren verbesserten mechanischen Eigenschaften, die auxetische Strukturen im Allgemeinen bieten, gehören eine hohe Eindring- / Schlagfestigkeit, eine geringere Ausbreitung von Ermüdungsrissen, eine größere Zähigkeit und Modulfestigkeit sowie eine Schwingungsdämpfung. Die hier offenbarten dehnbaren auxetischen Strukturen sind aufgrund dieser Eigenschaften potenziell interessant für militärische und zivile Anwendungen wie kugelsichere Westen und stoßfeste Fahrzeuge und Schiffe.

Elektronische Geräte, die an der Außenseite von architektonischen Gebäuden, Fahrzeugen, Schiffen, Flugzeugen oder im Inneren von Raketenschiffen angebracht werden sollen, müssen ebenfalls extrem verformungsfest sein. Viele dieser Anwendungen würden von dehnbaren auxetischen Substraten profitieren, da die hier vorgestellten Strukturen in einer 2D-Folie hergestellt werden können und sich um 3D-gekrümmte Oberflächen wie Kugeln, gekrümmte Rohre und Zylinder biegen können.

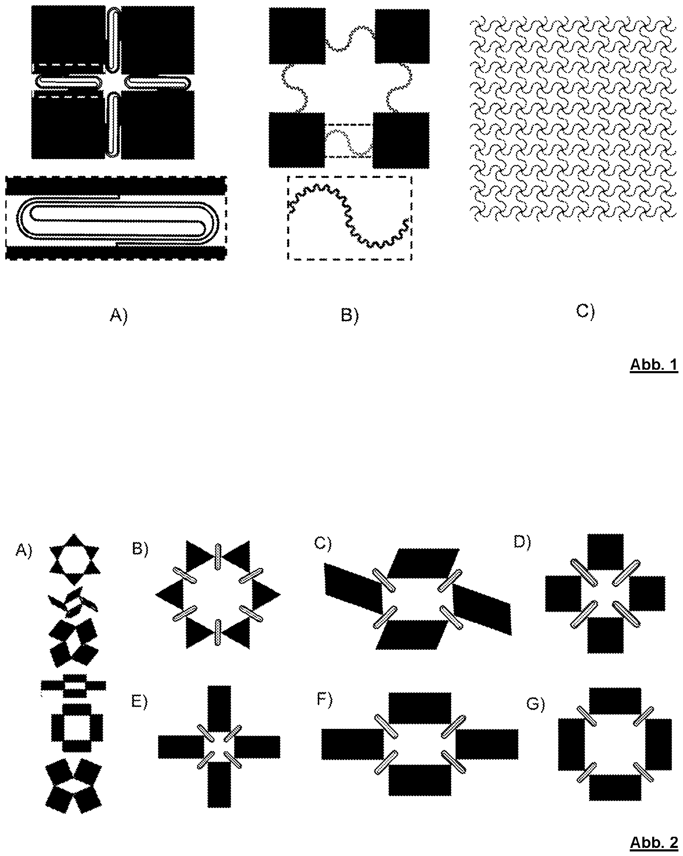

Ein Unterschied und ein Schlüsselmerkmal der Erfindung ist in der Tatsache zu sehen, dass herkömmliche dehnbare elektronische Strukturen, die im Insel-Verbindungsformat konfiguriert sind, eine Poissonzahl von Null haben, da sie sich in der Mitte der Insel verbinden, wie in der archimedischen Spiralverbindung in Abbildung 1A und in der fraktalen Hufeisen-Serpentinenverbindung in Abbildung 1B gezeigt wird.

Ein wesentlicher Unterschied und ein Hauptmerkmal der Erfindung kann auch darin gesehen werden, dass die Entwürfe des Standes der Technik zudem keine Inselstrukturflächen mit archimedischen Spiralverbindungen und / oder selbstähnlichen fraktalen Designverbindungen verbinden, um so durch die großen Inselstrukturflächen ausreichend Platz und Fläche für zukünftige Innovationen durch MEMS-Sensor/Aktor-Integration zu schaffen. Wie in Abbildung 1C zu sehen ist, bieten wellenförmige Maschenkonstruktionen nach dem Stand der Technik nicht genügend Platz für die Integration von Sensoren.

Ein weiterer Aspekt der vorliegenden Erfindung ist die Verbesserung der radialen Erweiterbarkeit der auxetischen Struktur, indem die starren Scharnierverbindungspunkte der rotierenden auxetischen Polygonstrukturen oder auch Einheitszellen durch klassische archimedische Spiralen und / oder selbstähnliche fraktale Designverbindungen ersetzt werden. Dieses Konzept wird durch die Einführung einer neuen Art der Darstellung, wie in den Abbildungen mit den Beispielen von archimedischen Spiralverbindungen gezeigt, deutlich. Durch die mechanische Gestaltung der geometrischen Eigenschaften der auxetischen Struktur, d. h. Oberflächendichte, archimedischer Spiralradius usw., können die Flexibilität / Dehnbarkeit der Struktur sowie der Bulkmodul, Schermodul, Elastizitätsmodul und die Poissonzahl des auxetischen Substrats eingestellt werden. Die Freiheit der geometrischen Gestaltung ermöglicht auch die Entwicklung von dehnbaren auxetischen Substraten mit hoher Flächendeckung, die in Zukunft Geräte mit höherem Wirkungsgrad ermöglichen könnten.

In der Literatur gibt es mehrere auxetische mechanische Metamaterialien, die so modifiziert werden können, dass sie spannungsarme Inselstrukturflächen enthalten. Einige Beispiele sind wabenförmige, chirale, re-entrante, Origami-basierte Metamaterialien, Kirigami-basierte Metamaterialien, Keyed-Brick-Strukturen, Strukturen mit Schlitzperforation, rotierende starre Strukturen und hierarchische auxetische Systeme.

Die erfindungsgemäße auxetische Struktur erfordert eine signifikante Modifikation der traditionellen auxetischen Strukturen aus der Literatur, indem deren starre oder rotierende Verbindungspunkte durch dehnbare Verbindungen ersetzt werden. Die mäanderförmigen wellenförmigen Verbindungen (Wickelbahnstrukturen) werden aus bekannten dehnbaren Verbindungen gebildet, wobei eine Ausführungsform der Erfindung auxetische Strukturen mit dehnbaren Verbindungen aus archimedischen Spiralstrukturen zeigt. Eine zweite Ausführungsform zeigt auxetische Strukturen mit dehnbaren fraktalen Verbindungen und eine dritte Ausführungsform zeigt auxetisches wellenförmiges Maschendesign. Um sicherzustellen, dass die geänderte Struktur ihr auxetisches Verhalten beibehält, muss die dehnbare Verbindung das auxetische Gerüst an der gleichen Stelle und im gleichen Winkel wie die früheren starren Verbinder durchschneiden. Das Ersetzen der starren Verbindungen durch dehnbare Verbindungen verbessert die mechanischen Eigenschaften der gesamten auxetischen Struktur erheblich und ermöglicht eine extreme Dehnbarkeit und Kompressibilität der Gesamtstruktur.

Die Erfindung wird dadurch erreicht, dass eine Anordnung von periodischen Inselstrukturflächen geschaffen wird, die auxetisch angeordnet sind, und dann die Inselstrukturflächen mit periodischen dehnbaren Zwischenverbindungen (z. B. mäanderförmige fraktalen Designverbindungen oder archimedische Spiralen) verbunden werden. Die so entstehenden dehnbaren auxetischen Strukturen können jedes beliebige Material in die Lage versetzen, extrem widerstandsfähig gegen angewandte Verformung zu werden.

Die Inselstrukturflächen in der Struktur erfahren geringe Spannungen unter angewandter Dehnung, wodurch sie sich großflächig für die Integration funktioneller elektronischer MEMS-Bauteile eignen. Diese Eigenschaft ist notwendig, um die Integration von drahtlosen Übertragungsantennen, Energy-Harvesting-Geräten, Energiespeichern und praktisch jeder Art von Sensor- oder Aktorkomponenten in medizinische Komponenten und Wearable Devices zu ermöglichen.

Das Design der Geometrie der dehnbaren Verbindungen (d. h. Amplitude, Wellenlänge, Dicke und Breite) erlaubt es, einen Kompromiss zwischen maximaler Flächendichte und Dehnbarkeit zu entwickeln. Großflächige, konforme, dehnbare auxetische Substrate würden MEMS/NEMS-Bauteile mit höherer Effizienz ermöglichen.

Alle oben genannten Anwendungen erfordern im Besonderen, dass das Substratmaterial mit traditionellen 2D-MEMS-Wafer-basierten Verarbeitungstechniken kompatibel ist.

Die offengelegten dehnbaren auxetischen Strukturen werden zahlreiche Anwendungen als Substrat / Rückgratstrukturen in den Bereichen dehnbare Elektronik, tragbare Elektronik, Stoßfestigkeit, energieabsorbierende Materialien, Architektur, Weltraumforschung und implantierbare medizinische Technologien haben. Wenn die Struktur als Substrat verwendet wird, profitiert jedes Material, das auf ein dehnbares auxetisches Substrat beschichtet wird, von den verbesserten mechanischen Eigenschaften, die durch die darunter liegende Struktur bereitgestellt werden.

Das hier weiter vorgestellte archetypische Design ermöglicht eine extreme Flexibilität und Dehnbarkeit innerhalb jeder Art von Gerät, was für medizinische Anwendungen der nächsten Generation wie intelligente Stents, intrasakkuläre Aneurysma-Geräte, medikamentenabgebende Implantate und tragbare Biosensoren von großer Bedeutung sein wird.

Formgedächtnislegierungen (SMAs), die in auxetischen Geometrien strukturiert sind, bieten überlegene mechanische Eigenschaften, die das Design von hochleistungsfähigen neuartigen medizinischen Implantaten mit erhöhter mechanischer Flexibilität und Dehnbarkeit ermöglichen können. SMAs können große intrinsische Dehnungen von bis zu 8 % durch eine thermische oder spannungsinduzierte Phasenumwandlung erreichen. Zudem werden SMAs in der diesseitigen Erfindung verwendet, weil sie mikrostrukturell so gestaltet werden können, dass sie eine (thermisch) spannungsinduzierte (mechanische) Phasenumwandlung für 10 Millionen Zyklen durchlaufen können, ohne ihre funktionalen Ermüdungseigenschaften zu verändern. Mit diesen Eigenschaften kann die Lebensdauer eines medizinischen Devices / Geräts erhöht werden und das Gerät so insgesamt sicherer für Patienten gemacht werden.

Die Erfindung hat auch Anwendungsmöglichkeiten auf dem Gebiet der Wearable Electronics. Beispielsweise müssen sich elektronische Tattoos und andere Substrate, die direkt auf der Haut getragen werden, natürlich an die Haut anpassen sowie über sehr große Strecken für Tausende oder Millionen von Zyklen reversibel dehnbar sein. Dank der synklastischen Biegung können sich auxetische SMAs besser an die Oberfläche der Haut oder des Substrats anpassen als dehnbare aktuelle Designs, während sie gleichzeitig eine größere Flächenabdeckung und eine bessere mechanische Leistung bieten als aktuelle Serpentinengitter-Designs. Dies könnte in Anwendungen nützlich sein, die Bio-Elektroden (Hautsensoren) erfordern, wie z. B. Elektrokardiogramm- und Magneto-Kardiogramm-Sensoren, die eine perfekte Schnittstelle zur Haut benötigen. Diese Anwendungen profitieren von der dezenten elektronischen Leitfähigkeit, die metallische SMAs bieten. Die überlegenen mechanischen Eigenschaften des SMA-Materials und die auxetischen Geometrien würden vielen Anwendungen im Zusammenhang mit dehnbarer und tragbarer Elektronik und weicher Robotik zugutekommen.

Nachfolgend wird die Erfindung anhand der beiliegenden Abbildungen in der **Abbildungsbeschreibung** beschrieben, wobei diese die Erfindung erläutern soll und nicht zwingend beschränkend zu werten ist. Es zeigen:
- Abb. 1: Übersicht über aus dem Stand der Technik bekannte dehnbare Geometrien mit Ähnlichkeiten zu der vorliegenden Erfindung;
- Abb. 2: Rotierende starre auxetische Strukturen gemäß dem Stand der Technik (Abb. 2A) und Ausführungsbeispiele für erfindungsgemäße rotierende archimedische Polygone (Abb. 2B-2G);
- Abb. 3: Finite-Elemente-Simulationen, die die verbesserte Dehnbarkeit und Kompressibilität einer beispielhaften erfindungsgemäßen rotierenden archimedischen Quadratstruktur zeigen (Abb. 3A-3G);
- Abb. 4: ein Ausführungsbeispiel einer erfindungsgemäßen rotierenden archimedischen quadratischen Anordnung (Ausschnitt - eine einzelne Einheit der archimedischen Spiralverbindung);
- Abb. 5: ein Beispiel für eine erfindungsgemäße rotierende archimedische Rechteckstruktur ausgebildet als 2D-Platte (Abb. 5A) und als 3D-Zylinder (Abb. 5B);
- Abb. 6: eine experimentelle Umsetzung einer erfindungsgemäßen rotierenden archimedischen Rechteckstruktur in Form eines 3D-Zylinders mit einem Durchmesser von 5 mm und einer Länge von 9 mm aus verschiedenen Perspektiven (Abb. 6A-6C);
- Abb. 7: eine experimentelle Umsetzung einer erfindungsgemäßen rotierenden archimedischen Rechteckstruktur geformt in eine 3D-Halbkugel mit einem Radius von 2,5 mm aus verschiedenen Perspektiven (Abb. 7A-7C);
- Abb. 8: Finite-Elemente-Simulationen und experimenteller Vergleich einer ersten beispielhaften erfindungsgemäßen rotierenden archimedischen Rechteck-Einheitszelle, die ein deutliches auxetisches Verhalten bei einachsigen Zug- und Druckprüfungen zeigt;
- Abb. 9: Finite-Elemente-Simulationen und experimenteller Vergleich eines zweiten Ausführungsbeispiels einer rotierenden archimedischen Rechteckstruktur, die ein deutliches auxetisches Verhalten zeigt;
- Abb. 10: eine Demonstration der elastischen und superelastischen Erholung von enormen Zug- und Druckbelastungen der in Abb. 8 und Abb. 9 gezeigten Ausführungsbeispiele auf der Grundlage von erfindungsgemäßen rotierenden archimedischen auxetischen Rechteckstrukturen;
- Abb. 11: Finite-Elemente-Simulationen der verstärkten Kompression in einer beispielhaften erfindungsgemäßen rotierenden archimedischen auxetischen Rechteckstruktur (Abb. 11A-11C);
- Abb. 12: Finite-Elemente-Simulationen der außerordentlichen Dehnbarkeit in einer beispielhaften erfindungsgemäßen rotierenden archimedischen Rechteckstruktur (Abb. 12A-12D);
- Abb. 13: ein Ausführungsbeispiel einer rotierenden rechteckigen auxetischen Struktur mit fraktalen Hufeisen-Serpentinen-Verbindungen zweiter Ordnung als 2D-Fläche (Abb. 13A), sowie die rotierende rechteckige auxetische Struktur mit fraktalen Hufeisen-Serpentinen-Verbindungen zweiter Ordnung in zylindrischer 3D-Form (Abb. 13B);
- Abb. 14: ein Ausführungsbeispiel einer rotierenden rechteckigen auxetischen Struktur mit fraktalen Hufeisen-Serpentinen-Verbindungen zweiter Ordnung und Inselstrukturflächen unterschiedlicher Größe als 2D-Fläche (Abb. 14A) sowie, die rotierende rechteckige auxetische Struktur mit fraktalen Hufeisen-Serpentinen-Verbindungen zweiter Ordnung und Inselstrukturflächen unterschiedlicher Größe geformt zu einer zylindrischen 3D-Geometrie (Abb. 14B);
- Abb. 15: Finite-Elemente-Simulationen, die das auxetische Verhalten der in Abb. 13 gezeigten beispielhaften erfindungsgemäßen Struktur basierend auf einer rotierenden rechteckigen auxetischen Struktur mit fraktalen Hufeisen-Serpentinen zweiter Ordnung bei Ausdehnung und Kompression belegen (Abb. 15A-15D);
- Abb. 16: ein Beispiel für ein 2D-Gitterdesign mit einer quadratischen Gittergeometrie unter Verwendung fraktaler Hufeisen-Serpentinen-Verbindungen zweiter Ordnung und quadratischer Inselstrukturflächen als 2D-Fläche (Abb. 16A), sowie das 2D-Gitterdesign mit einer quadratischen Gittergeometrie unter Verwendung von fraktalen Hufeisen-Serpentinen zweiter Ordnung und quadratischen Inselstrukturflächen geformt zu einer zylindrischen 3D-Geometrie (Abb. 16B);
- Abb. 17: Finite-Elemente-Simulationen, die das auxetische Verhalten mit verstärkter Ausdehnung und Kompression in dem in Abb. 16 gezeigten Ausführungsbeispiel einer Struktur im 2D-Gitterdesign unter Verwendung von fraktalen Hufeisen-Serpentinenverbindungen zweiter Ordnung und quadratischen Inselstrukturflächen zeigen (Abb. 17A-17D);
- Abb. 18: Finite-Elemente-Simulationen, die das auxetische Verhalten mit außerordentlicher Dehnbarkeit für das in Abb. 16 gezeigte Ausführungsbeispiel zeigen, bei dem eine einachsige Dehnung von einer Seite der Struktur aufgebracht wird und die andere Begrenzung als feste Einschränkung gehalten wird (Abb. 18A-18E);
- Abb. 19: Finite-Elemente-Simulationen, die das auxetische Verhalten mit erhöhter Kompressibilität für das in Abb. 16 gezeigte Ausführungsbeispiel zeigen, bei dem eine einachsige Druckbelastung von einer Seite der Struktur aufgebracht wird und die andere Begrenzung als eine feste Zwangsbedingung gehalten wird;
- Abb. 20: ein beispielhaftes Flussdiagramm mit den Schritten a) - d) zur Darstellung der 3D-Herstellung einer erfindungsgemäßen funktionalisierten Formgedächtnislegierung-Implantatstruktur;
- Abb. 21: ein beispielhaftes schematisches Flussdiagramm mit den Vorrichtungsquerschnittsschritten a) - e) für die Funktionalisierung einer erfindungsgemäßen implantierbaren auxetischen Struktur;
- Abb. 22: ein Beispiel entsprechend Abb. 21 Abschnitt d), ergänzt um wenigstens eine weitere Elektrode;
- Abb. 23: ein erstes Ausführungsbeispiel einer erfindungsgemäßen funktionalisierten implantierbaren Struktur (intelligenter Stent) in 3D-Röhrenform (Abb. 23A), im Ausschnitt die Einheitszelle der funktionellen auxetischen Struktur mit drei verschiedenen Gerätekomponenten (Abb. 23B) und eingebettet in ein Blutgefäß oder eine Arterie (Abb. 23C);
- Abb. 24: ein zweites Ausführungsbeispiel für eine "S-förmige-Inselstrukturflächen" auxetische Geometrie, so modifiziert, dass Inselstrukturflächen für die Integration von Geräten aus herkömmlichen "S-förmigen" auxetischen Strukturen zur Verfügung stehen, dargestellt als 2D-Fläche (Abb. 24A), als Ausschnitt (Abb. 24B) und als 3D intelligenter Stent (Abb. 24C);
- Abb. 25: Finite-Elemente-Simulationen des Ausführungsbeispiels einer "S-förmigen-Inselstrukturflächen" auxetischen Struktur aus Abb. 24 mit der Struktur im Gleichgewicht (Abb. 25A) und unter einachsiger Zugkraft (Abb. 25B) und
- Abb. 26: Darstellung des synklastischen Biegeverhaltens einer "S-förmigen Inselstrukturfläche" um eine 3D-Kugel, aufzeigend, dass diese als intrasakkuläre Vorrichtung verwendet werden könnte.

**Abb. 1** zeigt Beispiele für den Stand der Technik bei dehnbarer Elektronik. In Abb. 1A ist eine traditionelle Insel-Verbindungsstruktur mit archimedischen Verbindungen nach Jiang et al. ARCHIMEDEAN SPIRAL DESIGN FOR DEFORMABLE ELECTRONICS US 10,660,200 B2 (2020) dargestellt. Abb. 1B zeigt eine traditionelle Insel-Verbindungsstruktur mit fraktalen Hufeisen-Serpentinen-Verbindungen zweiter Ordnung und Abb. 1C zeigt ein 2D-Wellennetz mit hufeisenförmigen Bausteinen, die in einer quadratischen Gittergeometrie angeordnet sind. Gestaltungen gemäß Abb. 1B und 1C sind aus der Druckschrift Rogers et al. Self-similar and fractal design for stretchable electronics US 10,192,830 B2 (2019) bekannt.

Wie bereits zuvor dargestellt, sind dehnbare Inselverbindungen nach dem Stand der Technik typischerweise in der Mitte der Inseln miteinander verbunden, was zu einer Poissonzahl von Null unter einachsiger Belastung führt. Die Strukturen müssen biaxial (in x- und y-Richtung) gedehnt werden, um eine Ausdehnung zu erreichen, oder biaxial gestaucht werden, um eine vollständig zusammengedrückte Struktur zu erhalten. Bei einachsigen Zugversuchen (nur in y-Richtung) weisen die Strukturen eine positive oder eine Null-Poissonzahl auf. Das in Abbildung 1C vorgeschlagene 2D-Wellennetzwerk weist bekanntermaßen auxetische Eigenschaften bei einachsiger Dehnung auf. Der größte Nachteil dieses Designs besteht darin, dass keine großflächigen Inseln für die Integration funktionaler Bauteile zur Verfügung stehen.

**Abb. 2** zeigt in:
A) Beispiele für traditionelle im Stand der Technik bekannte auxetische Strukturen auf Basis rotierender starrer Strukturen beispielsweise bekannt aus Saxena, Krishna Kumar, Raj Das, and Emilio P. Calius. "Three decades of auxetics research- materials with negative Poisson's ratio: a review." Advanced Engineering Materials 18.11 (2016): 1847-1870
   und weiter in B) bis G) Ausführungsbeispiele für die erfindungsgemäßen auxetischen Strukturen mit erhöhter Dehnbarkeit unter Verwendung archimedischer Verbindungen, nämlich:
B) rotierendes archimedisches Dreieck
C) rotierendes archimedisches Parallelogramm
D) drehendes archimedisches Quadrat
E) drehendes archimedisches Rechteck Variante 1
F) drehendes archimedisches Rechteck Variante 2
G) drehendes archimedisches Rechteck Variante 3.

Die in dieser Erfindung vorgestellten Konstruktionen bieten ein extrem nachgiebiges und verformbares Substrat, das es jedem Material ermöglicht, flexibel, dehnbar und anpassungsfähig zu werden. In der Abbildung 2A sind im Stand der Technik bekannte Beispiele für traditionelle rotierende starre polygone auxetische Geometrien gezeigt. Abbildungen 2B - 2G zeigen Modifikationen der erfindungsgemäßen rotierenden starren auxetischen Strukturen mit archimedischen Spiralverbindungen, um eine verbesserte Dehnbarkeit und Kompressibilität zu ermöglichen. Hier wird die dehnbare archimedische Spirale mit den Inselstrukturflächen in demselben Winkel verbunden, in dem die Inselstrukturflächen zuvor in einer traditionellen rotierenden Polygonstruktur verbunden waren.

Tragbare Geräte, die direkt auf der Haut getragen werden sollen, müssen sich den extremen Bewegungen und den daraus resultierenden Veränderungen des Körperkrümmungsradius anpassen können. Dehnbare auxetische Strukturen müssen in der Lage sein, sich frei auszudehnen und zusammenzuziehen, um abrupte Verformungsänderungen auszugleichen.

Das auxetische Verhalten wird in den nachfolgenden Abbildungen für dehnbare auxetische Strukturen gezeigt, die auf rotierenden Quadraten gleicher Größe und rotierenden Rechtecken unterschiedlicher Größe basieren.

Finite-Elemente-Analyse (FEM) mit COMSOL Multiphysics 5.6 wird in einigen der nachfolgenden Abbildungen zur Darstellung des auxetischen Verhaltens der erfindungsgemäßen Strukturen unter einer einachsigen Druckkraft (gestaucht) und einer einachsigen Zugkraft (gedehnt) genutzt. Die dargestellte erfindungsgemäße Struktur weist eine negative Poissonzahl auf, wie die Volumenausdehnung in senkrechter Richtung bei einachsiger Verschiebung zeigt. Die maximale Dehnbarkeit der Einheitszelle hängt von der Gestaltung der beispielhaft verwendeten archimedischen Spiralverbindung ab.

Für alle vorgestellten FEM-Simulationsergebnisse wurde Kupfer als Material verwendet, wobei ein Elastizitätsmodul von E= 119 GPa, eine Poissonzahl von 0,34 und eine Dichte von 8940 kg/m3 angenommen wurde.

Die Analyse der von-Mises-Spannungsverteilung nach Anwendung der Dehnung auf die Einheitszelle bestätigt niedrige Spannungen auf den großen periodischen Inselstrukturflächen, da die Spannung in der gesamten duktilen Verbindung für alle modellierten Strukturen konzentriert und verteilt ist.

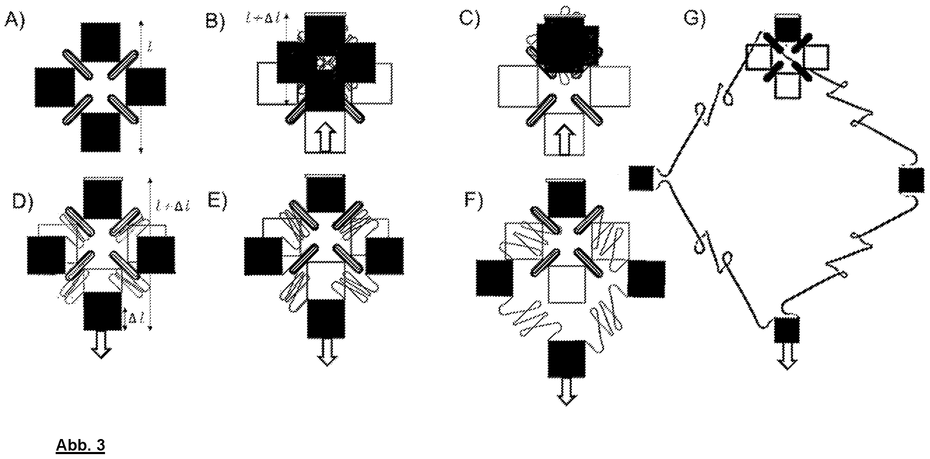

**Abb. 3** zeigt FEM-Ergebnisse, die das Ausmaß der verbesserten Kompressibilität und Dehnbarkeit für erfindungsgemäße rotierende quadratische auxetische Strukturen mit archimedischen Verbindungen verdeutlichen. Die Inselstrukturflächen sind 3,2 mm x 3,2 mm groß. Die archimedischen Spiralverbindungen haben eine Spitze-Spitze-Amplitude von 5 mm, eine Wellenlänge von 1,25 mm, eine Breite von 50 µm und eine Dicke von 50 µm. Die makroskopische einachsige Dehnung wird auf die unterste Kante der Struktur in der durch den Pfeil angegebenen Richtung angewendet.

Abb. 3A zeigt die Struktur unverformt im Gleichgewicht mit einem neutralen Abstand von Ende zu Ende = I. Der Betrag der makroskopischen Druck- oder Zugbelastung ist definiert als Längenänderung (ΔI) / Abstand von Ende zu Ende im Gleichgewicht (makroskopische Belastung % = ΔI/I * 100%).

Abb. 3B zeigt ein schwarzes Rechteck mit einer weißen Fläche auf einer Seite, um eine "feste Zwangsbedingung" an einer Kante des Quadrats darzustellen, während auf das gegenüberliegende Quadrat eine einachsige Auslenkung angewendet wird. Der große Pfeil zeigt an, dass die Richtung der aufgebrachten Kraft eine Druckkraft ist (in Richtung der festen Zwangsbedingung). Diese Abbildung zeigt das biaxiale Druckverhalten der Struktur bei einer einachsigen Druckbelastung von -30%. Der Betrag der makroskopischen Druck- oder Zugbelastung ist definiert als (ΔI/I).

Abb. 3C zeigt, dass sich die rotierende quadratische archimedische Struktur nach einer aufgebrachten Druckdehnung von -60 % aus der Ebene heraus verformt, so dass sich die Inselstrukturflächen der Struktur durch die Verformung aus der Ebene heraus fast übereinander stapeln.

Die Abb. 3D-3G zeigen die erfindungsgemäße auxetische Struktur bei verschiedenen makroskopischen uniaxialen Dehnungsgraden (~10% - 300%). Angezeigt durch den Pfeil, der von der festen Einspannung weg zeigt, wird ein Ende der auxetischen Struktur unter einachsiger Spannung gezogen. Die Struktur weist eindeutig eine negative Poissonzahl auf, die durch eine Ausdehnung in der senkrechten Richtung der aufgebrachten Zugkraft dargestellt wird.

Abb. 3G zeigt die Ausdehnung der Struktur um das Vierfache ihrer ursprünglichen Fläche (300 % der Gesamtdehnung der ursprünglichen Länge).

Die Entwicklung eines Arrays auf der Grundlage der vorgestellten beispielhaften erfindungsgemäßen Struktur würde ein hochgradig verformbares elektronisches 2D-Gerät ermöglichen, das sich im Wesentlichen an jede Art von konkaver oder konvexer 3D-Oberfläche anpassen kann.

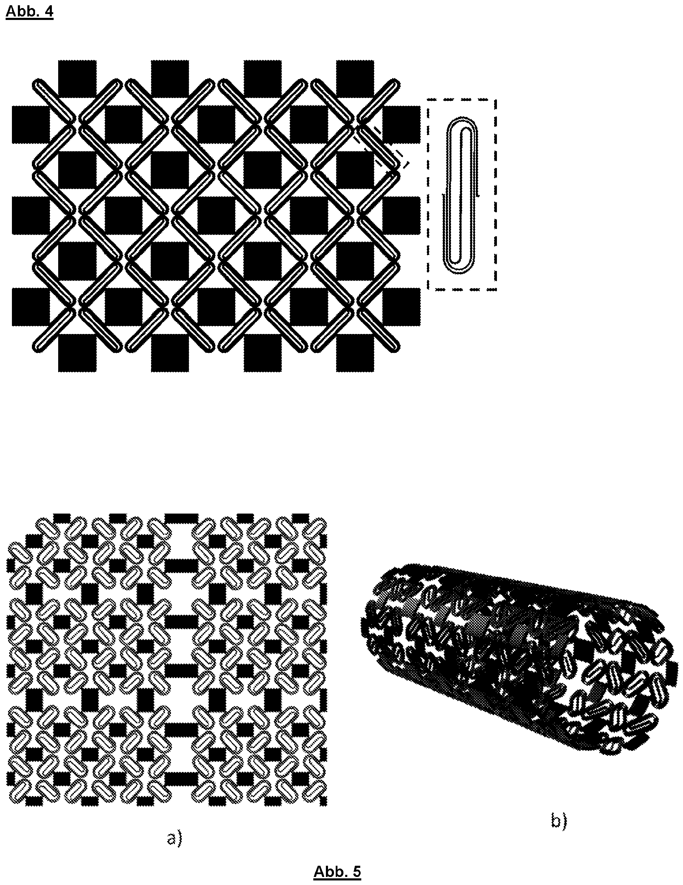

In **Abb. 4** wird ein Beispiel für eine rotierende quadratische archimedische Anordnung dargestellt. Der Ausschnitt zeigt dabei eine einzelne Einheit des archimedischen Spiralgestänges.

Ein Array einer rotierenden quadratischen archimedischen Polygonstruktur ist als Substrat für ein implantierbares medizinisches Gerät geeignet, da dies eine verbesserte Dehnbarkeit und Kompressibilität der Struktur erlauben würde, Crimp-Anforderungen zu erfüllen, um in den Körper durch einen Mikrokatheter eingeführt zu werden Für einen gegebenen Stentumfang bieten traditionelle auxetische Stentstrukturen 10-mal höhere Radialkräfte als aktuelle Designs (wie bspw. bekannt aus Dolla, William Jacob S., Brian A. Fricke, and Bryan R. Becker. "Structural and drug diffusion models of conventional and auxetic drug-eluting stents." (2007): 47-55.). Die Hinzufügung von gewundenen Stegstrukturen / Verbindungen, auch als dehnbare Interkonnektoren bezeichnet, zum Gerüst der auxetischen Strukturen reduziert die Biegesteifigkeit der Gesamtstruktur, so dass es bei kleinen angreifenden Kräften zu Quetschungen und Dehnungen kommt. Das bedeutet, dass medizinische Geräte, die auf der Basis von dehnbaren auxetischen Strukturen hergestellt werden, leichter zu crimpen, zu manövrieren und durch einen Mikrokatheter zu entfalten sind.

**Abb. 5** zeigt eine andere Anordnung der rotierenden archimedischen Rechteckstruktur mit unterschiedlich großen Rechtecken und einer archimedischen Spirale mit einer kleineren Spitze-Spitze-Amplitude (2,2 mm) im Vergleich zu der in Abb. 4 gezeigten Spirale.

Ein großer Vorteil der hier vorgestellten dehnbaren auxetischen Strukturen besteht darin, dass sie planar in 2D hergestellt werden können und sich dann an 3D-Oberflächen anpassen. Wenn die Struktur aus einer amorphen Formgedächtnislegierung besteht, ist es möglich, ein beispielsweise medizinisches Implantat vollständig mit Hilfe von 2D-MEMS-Techniken herzustellen und dann das Formgedächtnismaterial durch Kristallisation der Formgedächtnislegierung in eine komplizierte 3D-Geometrie zu bringen. Dieses Konzept wird in Abbildung 5B veranschaulicht, wo die 2D-Platte in eine röhrenförmige (oder zylindrische) 3D-Form gebracht wurde.

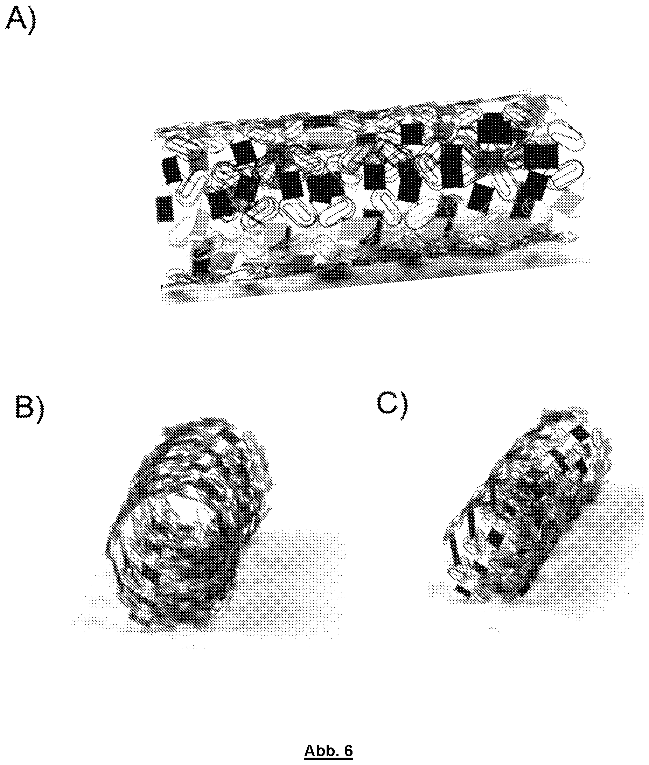

**Abb. 6** zeigt eine experimentelle Umsetzung einer erfindungsgemäßen rotierenden archimedischen auxetischen SMA-Rechteckstruktur aus TiNiCu (Dicke = 27 µm), die in eine röhrenförmige (oder zylindrische) 3D-Form mit einem Krümmungsradius von 2,5 mm und einer Länge von 9 mm gebracht wurde. Die Struktur könnte als intelligenter Stent zur Flussumleitung verwendet werden, wobei große Inselstrukturflächen für die elektronische Integration zur Verfügung stehen.

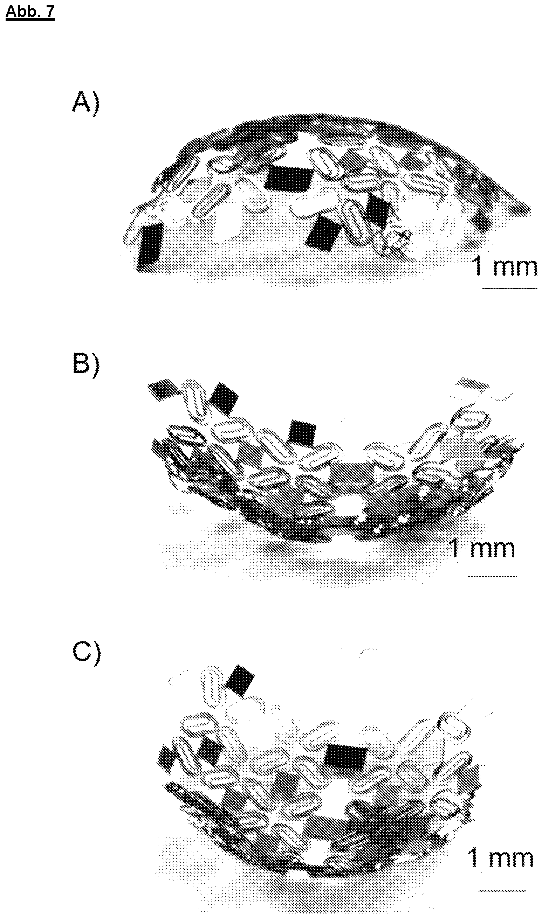

In **Abb. 7** wird eine experimentelle Umsetzung einer erfindungsgemäßen rotierenden archimedischen auxetischen SMA-Rechteckstruktur aus TiNiCuCo (Dicke = 53 µm) in einer 3D-Halbkugelgeometrie mit einem Krümmungsradius von 2,5 mm dargestellt. Diese Struktur könnte als funktionalisierte intrasakkuläre Aneurysma-Vorrichtung verwendet werden.

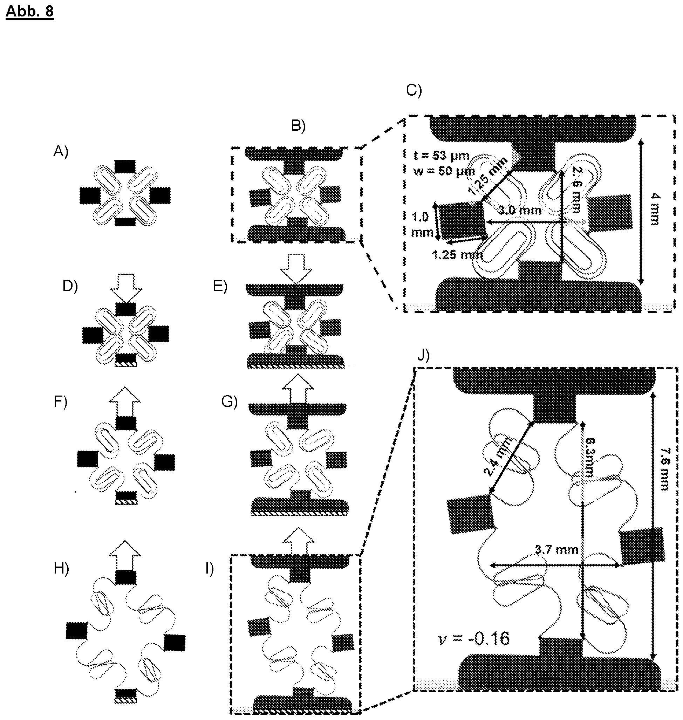

**Abb. 8** zeigt die verbesserte Dehnbarkeit und Komprimierbarkeit unter einachsiger Dehnung einer Einheitszelle mit rotierender archimedischer Rechteckgeometrie (Ausführungsbeispiel in Abbildung 5). Eine einachsige Dehnung wurde auf die oberste Kante der Struktur in der durch den Pfeil angegebenen Richtung ausgeübt, während die untere Kante der Struktur als feste Einspannung gehalten wurde (durch ein gestreiftes Rechteck gekennzeichnet). Die linke Spalte zeigt die Ergebnisse der Finite-Elemente-Modellierung des auxetischen Verhaltens unter Verwendung der Materialeigenschaften von Kupfer (Abb. 8A, D, F, H). Die mittlere Spalte zeigt experimentelle Ergebnisse eines rotierenden archimedischen Rechtecks, das aus der superelastischen Formgedächtnislegierung TiNiCuCo hergestellt wurde (Abb. 8B, E, G, I).

Formgedächtnislegierungen (SMAs) haben bekanntermaßen bessere mechanische Eigenschaften als herkömmliche Metalle wie Kupfer. SMAs sind das bevorzugte Substratmaterial für die in dieser Erfindung vorgestellten Strukturen. Die Formgedächtnislegierung NiTi ist biokompatibel und wird bereits in medizinischen Geräten wie Stents verwendet. Die Legierungen TiNiCu und TiNiCuCo sind nicht biokompatibel, haben sich jedoch bereits als SMAs mit extrem geringer Ermüdung erwiesen, die in der Lage sind, zwischen 0 % und 2 % intrinsischer Dehnung für mehr als 10 Millionen Zyklen zu zyklieren (Chluba, Christoph, et al. "Ultralow-fatigue shape memory alloy films". Science 348.6238 (2015): 1004-1007.). Neuartige dehnbare auxetische Strukturen wurden aus TiNiCuCo-Dünnschichten mit extrem geringer Ermüdung (Dicke = 53 µm) hergestellt, um den Nachweis des Konzepts zu erbringen.

In den Abb. 8A, B) zeigen die Finite-Elemente-Simulation (Abb. 8A) und das Experiment (Abb.8B) eine beispielhafte rotierende archimedische Rechteck-Einheitszelle im Gleichgewicht (der Abstand von Ende zu Ende (I) beträgt 4 mm). Die Einfügung in der rechten Spalte bezeichnet die Abmessungen der Inselstrukturflächen (1,0 mm x 1,25 mm) und der archimedischen Spirale (Wellenlänge (λ) = 1,25 mm, Spitze-Spitze-Amplitude (A) = 2,2 mm, Breite (w) = 50 µm, Dicke 53 µm) der Struktur im Gleichgewicht. Sowohl bei den Simulationen als auch bei den Experimenten wird davon ausgegangen, dass die äußersten Kanten der Geometrie im Gleichgewicht einen Abstand von 4 mm haben. Der Abstand zwischen den innersten Kanten der Rechtecke wird als Ausgangswert für die Berechnung der Poissonzahl verwendet (xGleichgewicht = 3,0 mm, yGleichgewicht = 2,6 mm).

In den Abb. 8D, E wird eine einachsige Druckgesamtdehnung von -10% auf die oberste Kante der Struktur aufgebracht, was zu Kontraktionsinselstrukturflächen in der senkrechten Richtung führt.

Die Abb. 8F, G zeigen eine einachsige Zugdehnung von 26,5% auf die oberste Kante der Struktur, was zu einer Ausdehnung der Inselstrukturflächen in der senkrechten Richtung führt.

Des Weiteren zeigen die Abb. 8H, I eine einachsige Zugbelastung von 90 % auf die oberste Kante der Struktur, was zu einer Ausdehnung der Inselstrukturflächen in die senkrechte Richtung führt. Bei einer Verschiebung von 7,6 mm in y-Richtung beträgt die Dehnbarkeit (Gesamtdehnung) der gesamten Einheitszellenstruktur 90 %. Die Einfügung zeigt, dass der Wert der Gesamtdehnung anhand des vertikalen Abstands zwischen den Außenkanten des oberen und unteren Quadrats ermittelt wird. Der Abstand zwischen den innersten Kanten der Rechtecke wird als Ausgangswert für die Berechnung der Poissonzahl verwendet (xstretched = 3,7 mm, ystretched = 6,3 mm).

In den Abb. 8C, J wird durch jeweilige Ausdehnung bzw. Stauchung in der senkrechten Richtung der aufgebrachten Kraft gezeigt, dass diese rotierende archimedische Rechteckstruktur tatsächlich auxetisch ist. Die Struktur wurde experimentell als auxetisch mit einer Poissonzahl von -0,16 nachgewiesen, die berechnet wurde, indem die Änderung der axialen Dehnung ((3,7mm - 3,0mm)/ 3,0mm) durch die Änderung der transversen Dehnung ((6,3mm - 2,6mm)/2,mm) dividiert wurde. Die Werte für diese Berechnung verwenden den inneren Abstand zwischen den Inselstrukturflächen, da die Dehnung der Spiralen für die Ausdehnung/Kompression der gesamten Strukturen verantwortlich ist. In den vier archimedischen Spiralen der Einheitszelle wurde während der Dehnungsexperimente eine ungleichmäßige Dehnung beobachtet, die zu Dehnungen zwischen 101 % und 189 % führte. Das ungleichmäßige Verhalten in den archimedischen Spiralen könnte auf 1) den Verbindungswinkel der Spirale mit den Inselstrukturflächen und 2) eine Fehlausrichtung der Struktur in der x-Achse bei der Durchführung von Zugversuchen parallel zur y-Achse zurückzuführen sein.

Alle in Abb. 8 dargestellten Maße und Werte sind exemplarisch für die beispielhafte dargestellte Anordnung der rotierenden archimedischen Rechteckstruktur aufgezeigt und sollen nicht beschränkend für unterschiedlich ausgeführte erfindungsgemäße Strukturen gewertet werden.

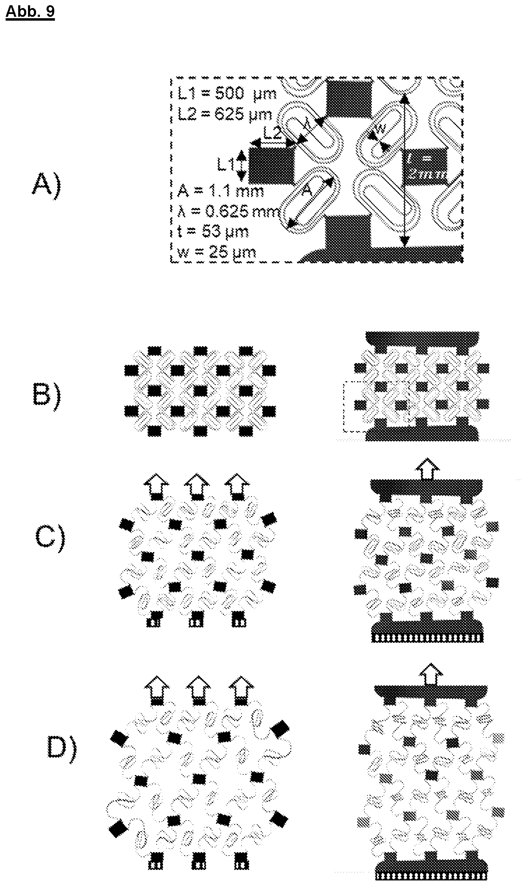

**Abb. 9** zeigt Finite-Elemente-Simulationen (FEM) und einen experimentellen Vergleich eines zweiten Ausführungsbeispiel einer rotierenden archimedischen Rechteckanordnung, bei der die Anordnung sowohl auxetisch als auch stark dehnbar ist. Mit Ausnahme der Dicke (t = 53 µm) wurden alle Abmessungen der in Abb. 8 gezeigten Einheitszelle um den Faktor 2 verkleinert (die Inselstrukturflächen sind 0,5 mm x 0,625 mm groß) und mit einer archimedischen Spirale versehen (Wellenlänge (λ) = 0,625 mm, Spitze-Spitze-Amplitude (A) = 1,1 mm, Breite (w) = 25 µm, Dicke 53 µm) und dann zu einem Array angeordnet (3 x 2), um die in Abb. 9 dargestellte Array-Struktur zu erzeugen. Die Simulationsergebnisse in der linken Spalte verwenden die Materialeigenschaften von Kupfer, während die experimentellen Ergebnisse in der mittleren Spalte wiederum die Formgedächtnislegierung TiNiCuCo verwenden.

Die untere Kante ist eine feste Einschränkung, während die obere Kante eine vorgeschriebene uniaxiale Verschiebung in Pfeilrichtung erfährt.

Abb. 9A) zeigt die gefertigte Struktur im Gleichgewicht mit Angabe aller relevanten Abmessungen für die Inselstrukturflächen (0,5 mm x 0,625 mm) und die archimedische Spirale (w = 25 µm, t = 53 µm, λ = 0,625 mm, A = 1,1 mm).

In Abb. 9B) werden Finite-Elemente-Simulation und Experiment der Struktur im Gleichgewicht (0% angewandte Dehnung) dargestellt.

Abb. 9C) zeigt FEM und Experiment der Struktur bei einer einachsigen 62%igen Zugbelastung vom oberen Rand aus
Zudem zeigt Abb. 9D) FEM und Experiment der Struktur bei einer einachsigen Zugbelastung von 102% ab Oberkante.

Alle in Abb. 9 dargestellten Maße und Werte sind exemplarisch für die beispielhafte dargestellte Anordnung der rotierenden archimedischen Rechteckstruktur aufgezeigt und sollen nicht beschränkend für unterschiedlich ausgeführte erfindungsgemäße Strukturen gewertet werden.

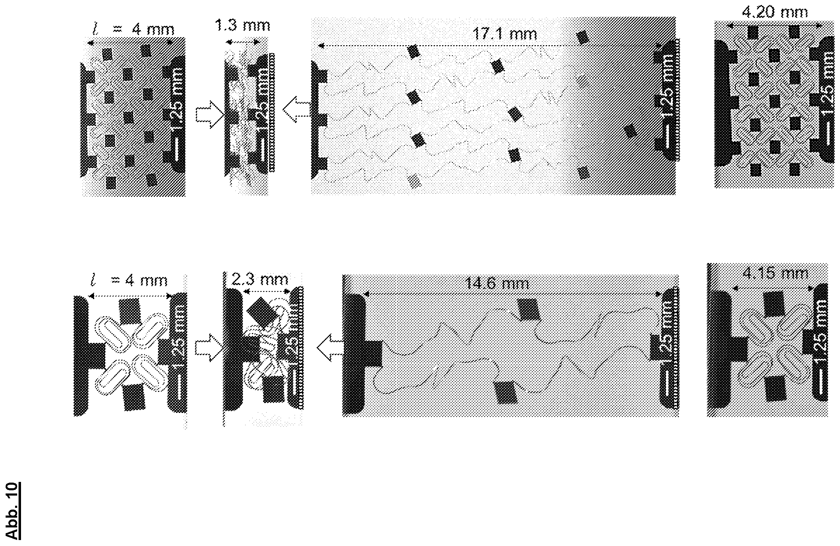

**Abb. 10** zeigt die experimentelle elastische Erholung von Riesendehnungen und - kompressionen der in Abb. 8 (rotierende archimedische Rechteck-Einheitszelle) und Abb. 9 (rotierende archimedische Rechteckanordnung) dargestellten Geometrien. Die elastische Erholung solch großer Gesamtdehnungen ist auf die Kombination aus der archimedischen Spirale und dem superelastischen Effekt zurückzuführen, der für TiNiCuCo-Formgedächtnislegierungen bekannt ist.

Abb. 10A) zeigt die rotierende archimedische Rechteck-Einheitszellenstruktur (linke Spalte) und angeordnete Struktur (rechte Spalte) im Gleichgewicht (0 % aufgebrachte Dehnung).

In Abb. 10B) werden beide Strukturen bei maximaler geprüfter Kompression von -47% (Einheitszelle) und -67% (Array) dargestellt.

Abb. 10C) stellt beide Strukturen bei einer maximal getesteten Dehnung von 265 % (Einheitszelle) und 330 % (Array) dar. Bei dieser angewandten Gesamtdehnung beträgt die maximale Dehnung in den archimedischen Spiralen 454 % (Einheitszelle) und 780 % (Array).

Eine superlastische Erholung der elastischen Dehnung nach Aufhebung der aufgebrachten Kraft mit extrem niedriger plastischer Gesamtdehnung auf die Strukturen von 3,75 % (Einheitszelle) und 5 % (Array) wird in Abb. 10D) dargestellt.

Die makroskopische Dehngrenze von Kupfer in den dehnbaren auxetischen Geometrien wird bestimmt, wenn die Fließspannung 200 MPa oder die erste Hauptdehnung 2 % übersteigt. Den Simulationsergebnissen zufolge können Kupferdünnschichten mit denselben Abmessungen wie die in Abb. 10 experimentell nachgewiesenen Einheitszellstrukturen die Einheitszelle nur um 15 % elastisch (Verschiebung um 0,6 mm) elastisch einachsig gedehnt werden, während TiNiCuCo um 265 % elastisch gedehnt werden kann. In ähnlicher Weise kann sich Kupfer in der Gitterstruktur elastisch bis zu einem Maximalwert von 112,5 % dehnen (Auslenkung von 4,08 mm), während TiNiCuCo sich elastisch um 330 % dehnen kann. Außergewöhnliche Dehnbarkeit wurde in der Formgedächtnislegierung archimedische Spirale mit einer fast vollständigen elastischen Erholung von einer Dehnung von 780% erreicht. Diese signifikanten Ergebnisse unterstützen die Annahme, dass Formgedächtnislegierungen ein bevorzugtes Material für dehnbare elektronische Substrate sind, die unter Verwendung der in dieser Erfindung offenbarten Strukturen hergestellt werden.

Alle in Abb. 10 dargestellten Maße und Werte sind exemplarisch für die beispielhafte dargestellte Anordnung der rotierenden archimedischen Rechteckstruktur aufgezeigt und sollen nicht beschränkend für unterschiedlich ausgeführte erfindungsgemäße Strukturen gewertet werden.

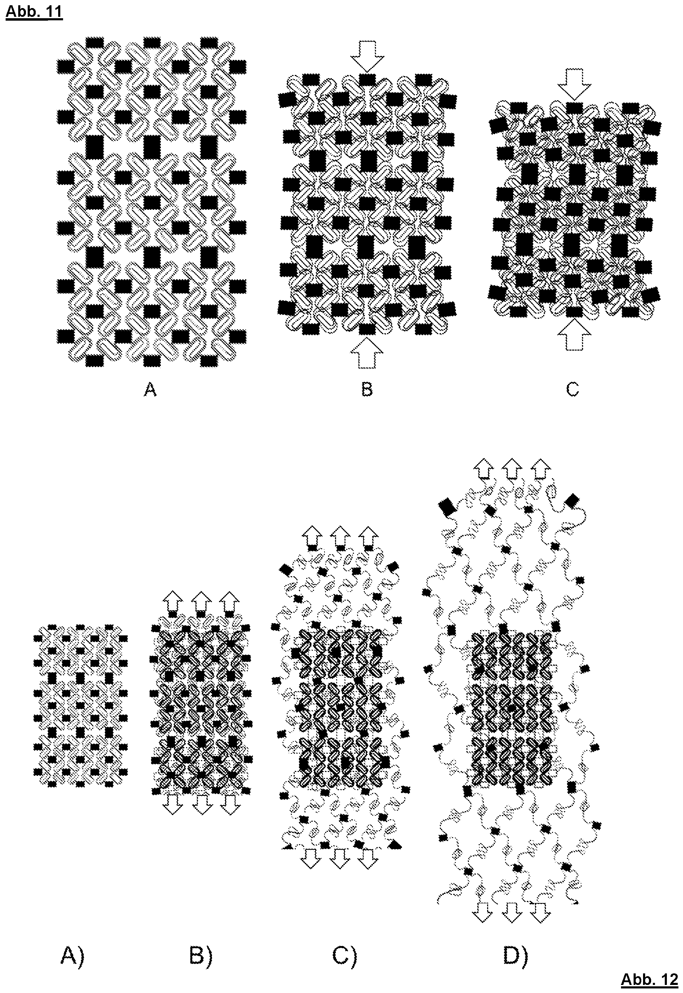

Für die Darstellung in den Abb. 11 und 12 wird die in Abb. 9 gezeigte erfindungsgemäße Struktur nochmals angeordnet (1x3), um eine größere rotierende archimedische Rechteckstruktur zu schaffen.

In **Abb. 11** werden beispielhafte FEM von einer verstärkten Kompression in einem rotierenden Rechteck mit archimedischer auxetischer Struktur (Abb. 11A (Gleichgewicht)-9C (stark komprimiert)) dargestellt. Hierzu wird auf die oberste und unterste Kante der Struktur eine einachsige Druckbelastung in den durch die Pfeile angegebenen Richtungen ausgeübt.

Abb. 11A) zeigt die Struktur im Gleichgewicht (0% Gesamtdehnung).

In Abb. 11B) wird die Struktur unter 10%iger Druckbelastung von der oberen Kante und 10%iger Druckbelastung von der unteren Kante, 20% einachsiger Druckbelastung insgesamt, gezeigt.

In Abb. 11C) zeigt die Struktur unter 16,66% Druckspannung vom oberen Rand und 16,66% Druckspannung vom unteren Rand. Das ergibt 33,33% einachsige Druckbelastung insgesamt.

**Abb. 12** zeigt die Simulationsergebnisse des auxetischen Verhaltens der Struktur unter uniaxialer Spannung. Es wird die einachsige Zugbelastung an der obersten und untersten Kante der Struktur in den durch die Pfeile angegebenen Richtungen dargestellt.

Abb. 12A) zeigt die Struktur im Gleichgewichtszustand (0 % aufgebrachte Dehnung).

In Abb. 12B) ist die Struktur unter 10 % Druckdehnung von der Oberkante und 10 % Druckdehnung von der Unterkante. Das ergibt 20% einachsige Druckdehnung insgesamt.

In Abb. 12C) ist die Struktur unter 50%iger Ausdehnung vom oberen Rand und 50%iger Ausdehnung vom unteren Rand dargestellt, was. 100% einachsige Zugdehnung insgesamt ergibt.

Abb. 12D) wiederum zeigt die Struktur unter 100%iger Ausdehnung von der Oberkante und 100%iger Ausdehnung von der Unterkante, also mit 200% einachsiger Zugdehnung insgesamt.

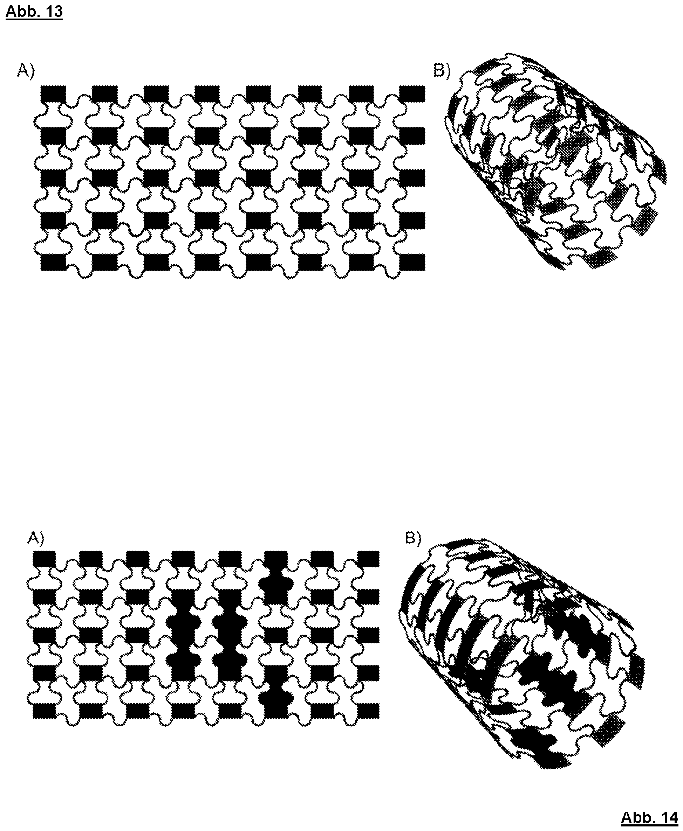

In **Abb.13** werden ein Ausführungsbeispiel, das eine rotierende rechteckige auxetische Struktur mit fraktalen Hufeisen-Serpentinen-Verbindungen zweiter Ordnung zeigt (Abb. 13A), sowie die rotierende rechteckige auxetische Struktur mit fraktalen Hufeisen-Serpentinen-Verbindungen zweiter Ordnung in zylindrischer 3D-Form (Abb. 13B) dargestellt.

Zudem werden in **Abb. 14** ein Ausführungsbeispiel, das eine rotierende rechteckige auxetische Struktur mit fraktalen Hufeisen-Serpentinen-Verbindungen zweiter Ordnung und Inselstrukturflächen unterschiedlicher Größe (Abb. 14A), sowie die rotierende rechteckige auxetische Struktur mit fraktalen Hufeisen-Serpentinen-Verbindungen zweiter Ordnung und Inselstrukturflächen unterschiedlicher Größe, die zu einer zylindrischen 3D-Geometrie geformt sind, gezeigt (Abb. 14B). In der Mitte der Struktur sind die Inselstrukturflächen mit einer anderen Größe und Form als am Rand der Struktur oder auch als in den vorangehenden Abbildungen dargestellt.

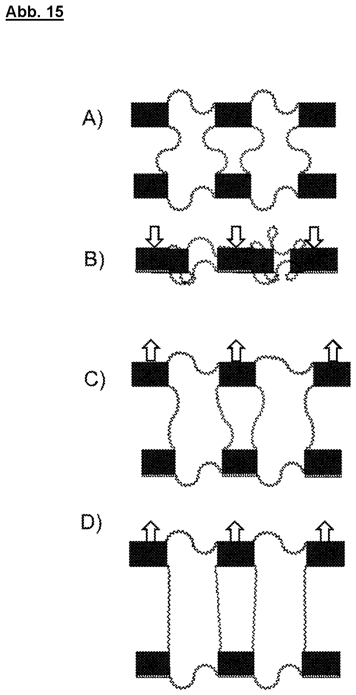

**Abb. 15** zeigt ein mögliches Ausführungsbeispiel der rotierenden rechteckigen auxetischen Struktur mit fraktalen Hufeisen-Serpentinen-Verbindungen zweiter Ordnung aus Abb. 13. Die unterste Kante wird als feste Einschränkung gehalten, während auf die oberste Kante eine einachsige Kraft ausgeübt wird. Abb. 15A) zeigt die Struktur im Gleichgewichtszustand. Abb. 15B) zeigt die Struktur unter 100% Druck. In Abb. 15C) ist die Struktur unter 40%iger uniaxialer Spannung und in Abb. 15D) unter 75%iger uniaxialer Spannung, wobei die Struktur auxetisches Verhalten aufweist.

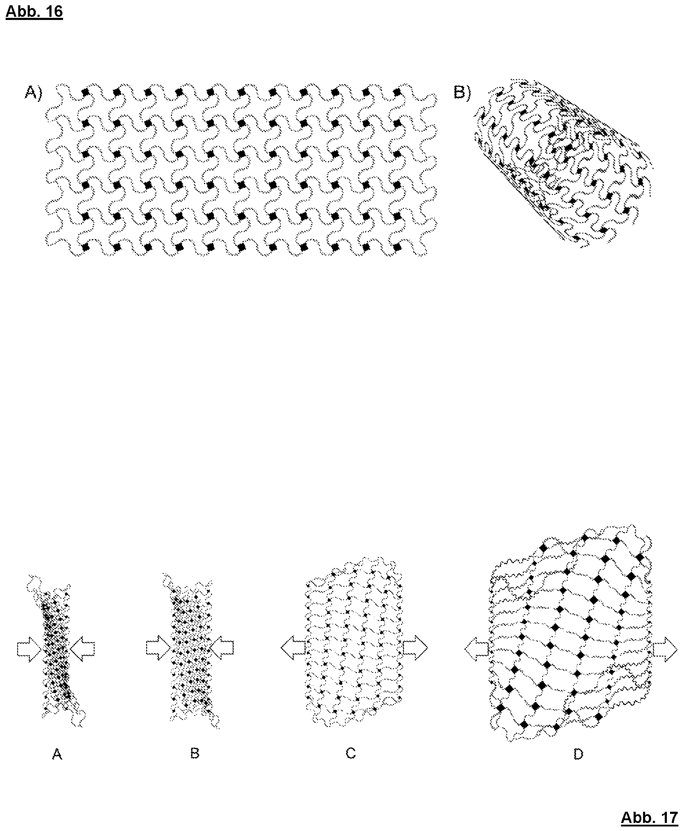

Beispiele für ein wellenförmiges 2D-Netzdesign mit einer quadratischen Gittergeometrie unter Verwendung fraktaler Hufeisen-Serpentinen-Verbindungen zweiter Ordnung und quadratischer Inselstrukturflächen und ein 2D-Wellengitterentwurf mit einer quadratischen Gittergeometrie unter Verwendung von fraktalen Hufeisen-Serpentinen zweiter Ordnung und quadratischen Inselstrukturflächen, die zu einer zylindrischen 3D-Geometrie geformt werden, werden in **Abb. 16** (Abb. 16A) - 16B)) dargestellt.

In **Abb. 17** wird das auxetische Verhalten einer Struktur im 2D-Wellengitterdesign mit quadratischer Gittergeometrie unter Verwendung von fraktalen Hufeisen-Serpentinenverbindungen zweiter Ordnung und quadratischen Inselstrukturflächen gemäß Abb. 14 mit verstärkter Ausdehnung und Kompression dargestellt. Die einachsige Dehnung wird sowohl auf die linke als auch auf die rechte Kante der Struktur in der durch den Pfeil angegebenen Richtung ausgeübt. Es zeigen:
17A) Struktur unter 30%iger Druckbelastung von der Oberkante und 30%iger Druckbelastung von der Unterkante. Einachsige Druckdehnung von insgesamt 60%.
17B) Struktur unter 20% Druckbelastung von der Oberkante und 20% Druckbelastung von der Unterkante. Einachsige Druckdehnung von insgesamt 40%.
17C) Struktur unter 10% Dehnung vom oberen Rand und 10% Dehnung vom unteren Rand. 20% einachsige Zugdehnung insgesamt.
17D) Struktur unter 30% Ausdehnung von der Oberkante und 30% Ausdehnung von der Unterkante. Einachsige Zugdehnung von insgesamt 60%.

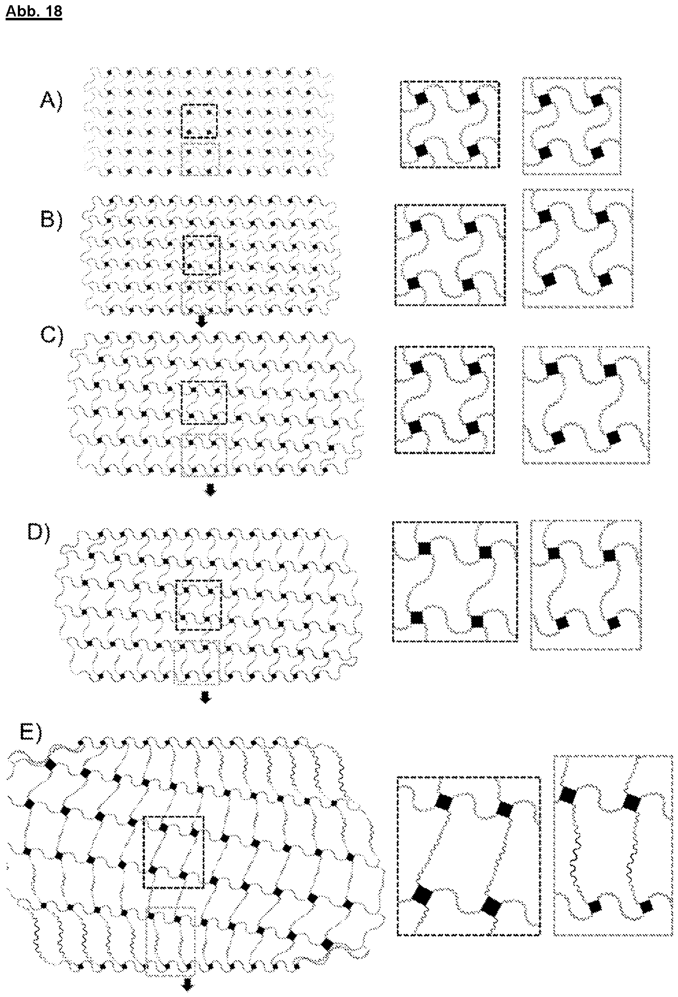

In **Abb. 18** wird das auxetische Verhalten mit erhöhter Dehnbarkeit in einem 2D-Wellengitter mit quadratischer Gittergeometrie unter Verwendung fraktaler Hufeisen-Serpentinenverbindungen zweiter Ordnung und quadratischer Inselstrukturflächen demonstriert. Der schwarze Einsatz zeigt die innerste Einheitszelle der Gitterstruktur, während der graue Einsatz die Randeinheitszelle zeigt, die der Richtung der aufgebrachten Kraft der Gitterstruktur unter Dehnung am nächsten liegt. Die oberste Kante der Struktur wird als feste Einschränkung gehalten, während die untersten Kanten der Struktur in der durch den Pfeil angegebenen Richtung einachsig gedehnt werden. Es werden ein ungleichmäßiges Dehnungsverhalten und eine Biegung außerhalb der Ebene beobachtet.
18A) Struktur im Gleichgewichtszustand
18B) Struktur unter ~5% Ausdehnung
18C) Struktur bei ~10% Ausdehnung
18D) Struktur unter ~20% Ausdehnung
18E) Struktur unter ~100% Ausdehnung

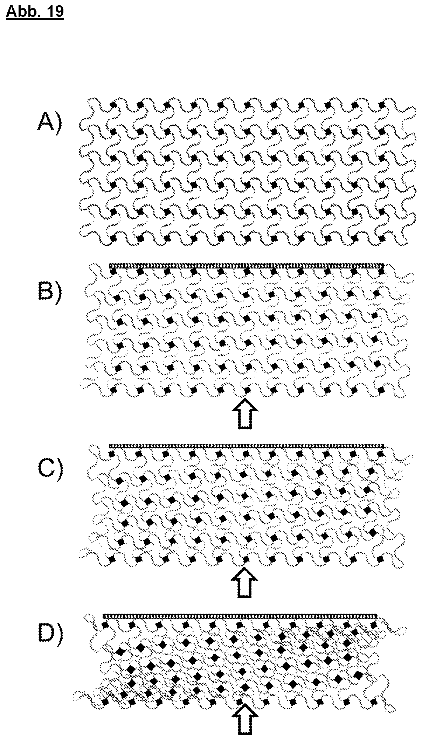

**Abb. 19** stellt eine Finite-Elemente-Simulation des auxetischen Verhaltens mit erhöhter Kompressibilität in einem 2D-Wellengitter mit quadratischer Gittergeometrie unter Verwendung von fraktalen Hufeisen-Serpentinenverbindungen zweiter Ordnung und quadratischen Inselstrukturflächen dar. Eine einachsige Druckbelastung wird auf die untersten Kanten der Stegstruktur in der durch den Pfeil angegebenen Richtung ausgeübt. Es wird ein ungleichmäßiges Dehnungsverhalten beobachtet. Es zeigen:
19A) Struktur im Gleichgewichtszustand (0% Belastung)
19B) Struktur unter ~10% Kompression
19C) Struktur unter ~20% Kompression
19D) Struktur unter ~40% Kompression.

Dünne Filme aus auxetischen Formgedächtnislegierungen werden das bevorzugte Trägermaterial für künftige medizinische Implantate sein. Formgedächtnislegierungen wie TiNi werden derzeit in passiven medizinischen Geräten wie Stents verwendet, weil sie biokompatibel sind und klein genug gemacht werden können, um über einen Mikrokatheter in die Arterien des Körpers eingeführt zu werden. Der Hauptvorteil der Verwendung von Formgedächtnislegierungen als funktionalisiertes auxetisches Grundgerüst für medizinische Implantate besteht darin, dass sie dem Implantat zusätzliche Funktionalität verleihen (d.h. Selbstentfaltung, Formerholung, Superelastizität). Große Dehnungen von 6-8 % werden durch thermische und spannungsinduzierte Phasenumwandlungen erreicht, daher können sich auxetische SMAs auf viel größere Volumina ausdehnen oder dekonvolvieren als herkömmliche auxetische Metalle (beispielsweise Stahl). Darüber hinaus können nicht-biokompatible Formgedächtnislegierungen auf NiTi-Basis wie TiNiCu und TiNiCuCo so entwickelt werden, dass sie ein extrem niedriges Ermüdungsverhalten aufweisen, das es ihnen ermöglicht, ~10 Millionen Übergangszyklen mit minimaler Veränderung des mechanischen oder thermischen Verhaltens zu durchlaufen, was sie für tragbare Geräte attraktiv macht.

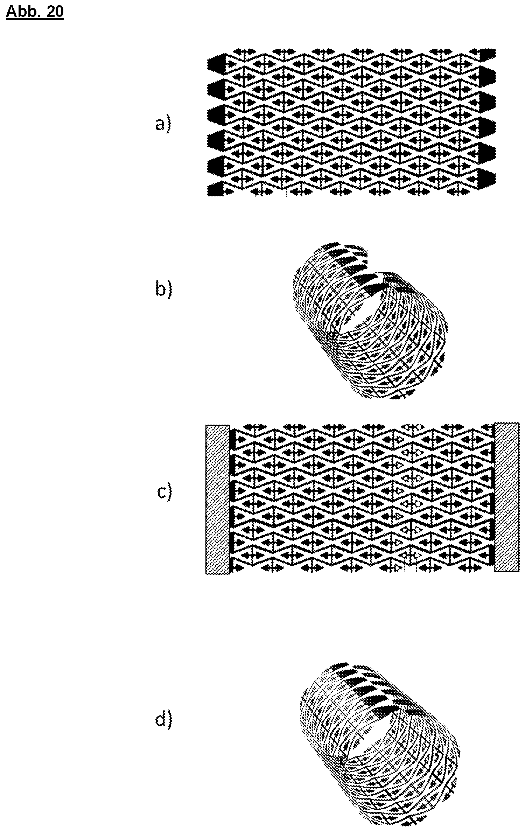

In **Abb. 20** wird die Herstellung eines funktionalisierten intelligenten Stents unter Verwendung eines auxetischen Substrats aus einer Formgedächtnislegierung dargestellt. Abb. 20a) zeigt, dass das Ausgangsmaterial eine amorphe auxetische Dünnschicht-SMA-Struktur ist, die in einem flachen 2D-Zustand durch eine aus dem Stand der Technik bekannte Herstellungsmethode (z. B. Photolithographie oder Laserschneiden) hergestellt wird. Die typische reentrant auxetische Geometrie wurde so modifiziert, dass sie pfeilförmige Inselstrukturflächen mit geringer Spannung aufweist, die wir "reentrantische Pfeil-Insel-Strukturen" nennen. Abb. 20b) zeigt, dass das amorphe Blech durch Kristallisation bei hoher Temperatur (400°C-800°C) in eine komplexe 3D-Form gebracht werden kann, z. B. durch schnelles thermisches Glühen um ein Objekt aus rostfreiem Stahl in der gewünschten Form. Abb. 20c) zeigt, dass die kristallisierte auxetische 3D-Struktur durch Anwendung mechanischer Kraft (d. h. durch Festklemmen des Bauteils auf einem ebenen Substrat) wieder in einen flachen 2D-Zustand gebracht werden kann. An diesem Punkt ist das Substrat bereit für die typische fortschrittliche Mikrobearbeitung zur direkten Herstellung und Integration von MEMS- und NEMS-Komponenten auf den Inselstrukturflächen der auxetischen Struktur.

Da das SMA-Grundgerüst leitfähig ist, können beide Seiten des SMA-Substrats funktionalisiert werden, was die verfügbare Oberfläche für die Integration von Bauteilen vergrößert. Da das SMA eine gemeinsame Masseelektrode sein kann, kann es als "Leiterplatte" fungieren. Beispiele für Bauelemente, die sich für eine Funktionalisierung eignen, sind integrierte Biosensoren und Auslesekomponenten, Antennen, piezoelektrische Bauelemente, Batterien, Kameras, LEDs, Aktoren und dergleichen. Abb. 20d) zeigt, dass nach der Herstellung aller funktionalen Bauelemente die mechanische Klammer entfernt wird, so dass das funktionalisierte auxetische Formgedächtnissubstrat seine 3D-Form elastisch wiedererlangen kann, wenn es mit einem superelastischen SMA hergestellt wurde. Da auf den Inselstrukturflächen bei Biege-, Streck-, Dreh- und Faltbewegungen nur geringe Spannungen auftreten, profitieren auch die auf den Inselstrukturflächen des SMA-Substrats strukturierten integrierten Schaltkreise von geringen Spannungen bei der Verformung. Liegt die Austenit-Endtemperatur über der Raumtemperatur, muss das funktionalisierte medizinische Implantat erhitzt werden, um seine ursprüngliche Form vollständig wiederzuerlangen. Da die Phasenumwandlung reversibel ist, können der Formgedächtniseffekt und der superelastische Effekt genutzt werden, um das Bauteil auf das erforderliche kleine Volumen des Mikrokatheters zusammenzudrücken und es dann durch Erhitzen auf Körpertemperatur (~37°C) in seine endgültige Position zu bringen.

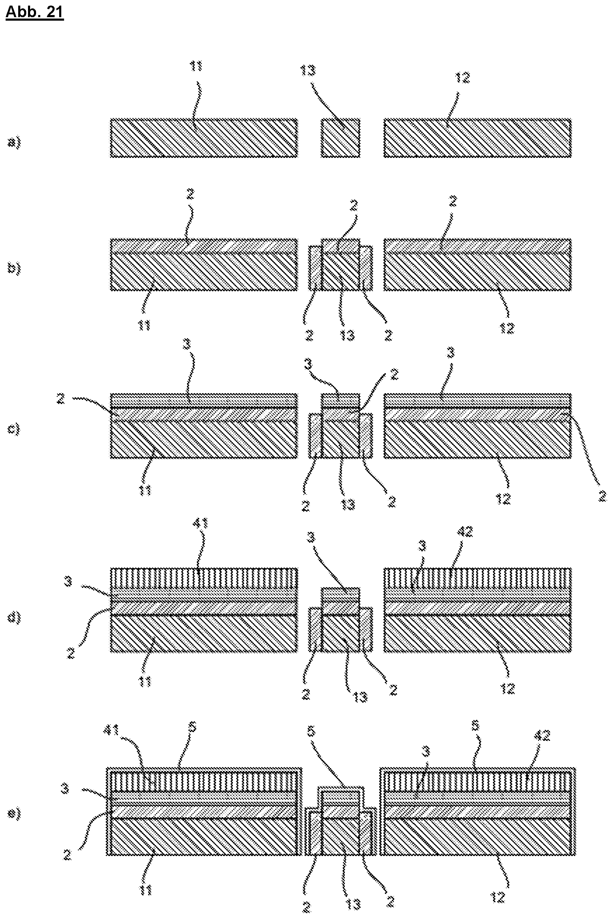

**Abb. 21** zeigt den abschnittsweisen Aufbau einer erfindungsgemäßen auxetischen Steg- oder Feldstruktur mit Verbindungen 13 und Freiräumen und Inselstrukturflächen 11, 12 als implantierbare Struktur aus einem biokompatiblen Material im Querschnitt.

Es handelt sich hierbei um ein Beispiel für eine Reihe von Fertigungsschritten, die zur Herstellung und elektrischen Isolierung zweier unterschiedlicher Bauelemente auf benachbarten Inselstrukturflächen durchgeführt werden können, nachdem das auxetische SMA-Grundgerüst mechanisch flach geklemmt wurde, wie in Abb. 20c) beschrieben.

In Schritt a) wird zunächst die aus einer kristallisierten Formgedächtnislegierung / SMA / shape memory alloy bestehende auxetische Grundstruktur hergestellt, wobei hierbei Inselstrukturflächen 11, 12 und Verbindungen 13 ausgebildet werden, wobei die Verbindungen die Inselstrukturflächen miteinander verbinden. Es sind in diesem Ablauf- bzw. quasi Herstellungsschema exemplarisch nur zwei Inselstrukturflächen 11,12 dargestellt, die dann funktionalisiert werden. Einige Inselstrukturflächen können hierbei entsprechend funktionalisiert sein bzw. im Weiteren funktionalisiert werden.

In Schritt b) wird eine erste elektrische Isolationsschicht 2 auf zumindest die Bereiche der Struktur aufgebracht, in denen elektrische Verbindungen und / oder elektrische Komponenten / Elektronikkomponenten aufgebracht werden sollen. Es kann auch ein vollständiger Überzug der Struktur erfolgen.

In Schritt c) erfolgt der Auftrag einer ersten Elektrode 3, so dass zumindest die notwendigen Bereiche mit einem entsprechenden leitfähigen Material überzogen sind. Also zumindest die Bereiche der Verbindungen und derjenigen Inselstrukturflächen, die eine elektrische Konnektierung benötigen werden mit der ersten Elektrode 3 belegt.

Durch diese Ausgestaltung ist bereits eine elektrische Struktur realisiert, da die Grundstruktur, nämlich die metallische auxetische SMA-Struktur (mit der Formgedächtnislegierung) 11, 12, 13 als gemeinsame Masseelektrode dient und die nun aufgebrachte Elektrode 3 den weiteren Leiter darstellt. Das SMA auxetische Rückgrat ist selbst sodann die gemeinsame Masseelektrode, um so elektrische Komponenten zu verbinden.

In Schritt d) erfolgt dann der Aufbau der Elektronikkomponenten 41, 42, die unterschiedlicher Natur sein können bzw. sind und insbesondere in Kombination zu einem großen System zusammengeschaltet werden können.

In Schritt e) erfolgt dann als letzter Schritt in diesem Beispiel der Überzug der Gesamtstruktur mit einem biokompatiblen Material. Beispielsweise kann dies ein biokompatibles Polymer sein, das den menschlichen Körper vor Elektronik schützt. Der Bereich der metallischen auxetischen SMA Struktur kann hierbei ausgespart werden, da dieser biokompatibel ist.

An dieser Stelle sei angemerkt, dass durch ein sinnvolles Strukturieren nicht notwendigerweise alle Bereiche der auxetischen SMA-Struktur 11, 12, 13 mit entsprechenden Schichten und/oder Bauteilen versehen werden müssen, sondern es kann leere Inselstrukturflächen und Verbindungen geben, die eine stützende Aufgabe haben, jedoch keine Tragfunktion für elektrische Komponenten und auch keine elektrische Leitung übernehmen. Dies unterscheidet sich von den Methoden in der Druckschrift US 2020/0144431 A1, bei denen ein Halbleiterbauelement auf jeder Inselstrukturfläche und jeder Serpentine einer auxetischen Gitterstruktur mit Serpentinen-Verbindungen strukturiert werden musste.

Nicht weiter dargestellt, aber zudem auch möglich, ist das Positionieren der ersten Elektrode oberhalb der elektrischen Komponenten bzw. Elektronikbauteile, so dass auf die auxetische SMA-Struktur zunächst eine elektrische isolierende Schicht aufgebaut wird und dann die Elektronikkomponenten bzw. allgemein die Bauteile aufgebracht werden, im Anschluss gefolgt von einer weiteren elektrisch isolierenden Schicht gefolgt von der Elektrode. Es können zudem weitere Leiter, jeweils getrennt durch elektrische isolierende Schichten aufeinander aufgebaut werden. Konstruktiv bedingte Abweichungen hiervon sind natürlich möglich.

Nach Abschluss der Herstellung aller MEMS-Komponenten wird das SMA über seine martensitische Phasenumwandlungstemperatur (in der Regel Körpertemperatur ~37 °C) erhitzt, um die Form wiederherzustellen, die zuvor beim Glühen festgelegt wurde. Wenn das Bauteil als medizinisches Implantat vorgesehen ist, kann es nun gekrimpt werden, um in einen Mikrokatheter eingesetzt zu werden. Wurde an eine flache 2D-Form gedacht, ist das funktionalisierte dehnbare / flexible Bauteil bereit, sich an das darunter liegende Substrat anzupassen (z. B. an die Haut eines tragbaren E-Tattoos oder Biosensors). Die funktionalisierten Geräte können in diesem Stadium optional in ein biokompatibles Polymer eingebettet (oder beschichtet) werden, falls dies für die Anwendung erforderlich ist.

Zum Auftragen der unterschiedlichen funktionalen Schichten sind verschiedene im Stand der Technik bekannte Dünnschichtverfahren anzuwenden. Hierzu sei bevorzugt auf das Sputtern, insbesondere Magnetron-Sputtern hingewiesen. Weitere Dünnschichtverfahren sind jedoch ebenso sinnvoll möglich, wie beispielsweise Sol-Gel oder Vapour-Dispositionsverfahren. Es sei zudem auch auf die Druckschrift US 8,758,636 B2 hingewiesen, die ein Verfahren zur Herstellung eines medizinischen Funktionselements mit einer selbsttragenden Gitterstruktur offenbart, wobei hier gezielt auf die Herstellung von speziellen Komponenten aus einer Formgedächtnislegierung mit dünner Folie beschrieben wird, die für Stentvorrichtungen vorgesehen sind.

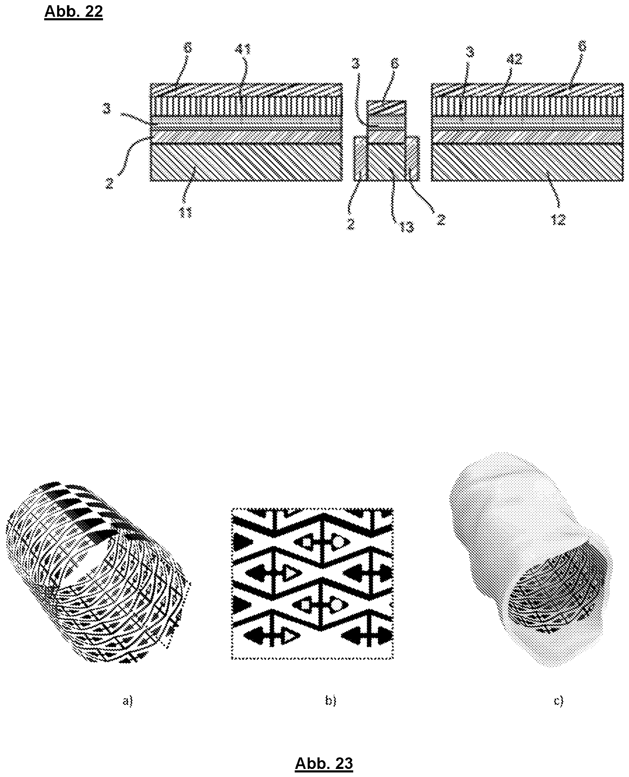

**Abb. 22** zeigt die aus Abb. 21 Abschnitt d) bekannte Struktur, ergänzt um wenigstens eine weitere Elektrode 6. Diese Elektrode 6 kann zusätzlich weitere Funktionen der Elektronikkomponenten übernehmen, beispielsweise eine Kommunikation oder zusätzliche Übertragung von Energie oder dgl. Es können auch noch weitere Elektroden vorgesehen werden, wobei diese dann unterschiedliche Elektronikkomponenten miteinander verbinden können.

Es können komplexe Netzstrukturen aufgebaut werden.

In **Abb. 23** wird eine erste beispielhafte Ausführungsform einer erfindungsgemäßen implantierbaren Struktur in Form einer Röhre, die in einer Arterie eingesetzt wird unter Verwendung einer modifizierten reentrant auxetischen Geometrie, wie in Abb. 20 bereits eingeführt, dargestellt.

Abb. 23a) zeigt eine reentrantische auxetische Pfeilinsel-Struktur in einer 3D-Zylindergeometrie mit spannungsarmen Inselstrukturflächen (in Form von Pfeilen), die mit MEMS-Komponenten gemäß dem in Abb. 20 vorgestellten Prozessablauf funktionalisiert sind.

Diese Ausführungsform kann als intelligenter Stent verwendet werden, wobei der 2D-Ausschnitt in Abb. 23b drei verschiedene elektronische Bauteile (dargestellt durch ein Quadrat, einen Kreis und ein Dreieck) zeigt, die auf den pfeilförmigen Inselstrukturflächen der modifizierten auxetischen Struktur mit geringer Spannung angebracht sind. Abb. 23c) zeigt das Bauteil, das in der Arterie eingesetzt wird. Das auxetische Grundgerüst des intelligenten Stents ermöglicht einen konformen Kontakt mit der nichtlinear gekrümmten Oberfläche der Arterie. Medizinische Implantate der nächsten Generation müssen mit einer Reihe von elektronischen Geräten ausgestattet sein, die lebenswichtige Patientendaten messen und diese in Echtzeit an den Arzt übermitteln. Ein funktionsfähiger kardiovaskulärer Stent könnte zum Beispiel den Blutdruck eines Patienten sowie viele andere Vitalparameter von zu Hause aus überwachen und die Daten dann drahtlos an das Krankenhaus oder einen Arzt übertragen.

Auxetische Formgedächtnislegierungen wären besonders attraktiv für den Einsatz in medizinischen Implantaten und Stents in Arterien des kardiovaskulären und neurologischen Systems, da die Implantate dem Patienten durch nicht-invasive endovaskuläre Behandlungen zugeführt werden könnten. Die negative Poissonzahl ermöglicht es auxetischen Strukturen, ihre Form zu verändern und sich der umgebenden Oberfläche, den komplexen Arterien und Venen des Körpers, anzupassen, wie in Abb. 23c) dargestellt. Nachdem ein Stent mehrere Jahre lang im Körper eines Patienten platziert wurde, kann es vorkommen, dass der Stent große und kleine Blutgerinnsel einschließt. Im Laufe der Zeit kann dies zu einer vollständigen Verstopfung der Arterien führen und ernste und lebensbedrohliche medizinische Komplikationen wie eine Lungenembolie, einen Herzinfarkt oder einen Schlaganfall verursachen. Die Ansammlung von körpereigenem Material (z. B. Gewebe, Zellen) auf dem Stent könnte mit einem Kraftsensor in einem intelligenten auxetischen Stent überwacht werden. Eine Antenne könnte ein Signal aussenden, um den Arzt zu benachrichtigen, wenn eine kritische Schwelle erreicht ist. Der funktionelle Stent könnte sich auch selbst mit Energie versorgen, wenn ein piezoelektrischer Energiekollektor in die Insel oder das Rückgrat der auxetischen Struktur integriert wird.

Darüber hinaus ist es möglich, MEMS- und NEMS-Komponenten direkt herzustellen, um die Inselstrukturflächen der auxetischen Struktur mit integrierten Sensoren und Auslesekomponenten zu versehen.

Als weiteres Beispiel könnte das hier Offengelegte eine Plattform für die Integration von Komponenten wie Kameras, Lichter (LEDs) und Sensoren zur Führung / Positionierung / Verfolgung der Stentposition während der Platzierung darstellen. Mit weiteren Innovationen dieser Erfindung könnte dies eine Methode sein, die in Zukunft die Notwendigkeit von MRT- oder Röntgenaufnahmen für die Stentplatzierung überflüssig macht. Zusätzlich zur Anpassung an die Form des Körpers kann die auxetische Struktur zudem so gestaltet werden, dass ein Kompromiss zwischen der Flächendichte der Einheitszelle und der maximalen Volumenausdehnung der gesamten Struktur erreicht werden kann. So können beispielsweise Änderungen des Blutflusses (Drucks) in einigen Arterien durch die Gestaltung der Flächendichte der auxetischen Struktur (d. h. große vs. kleine Inselstrukturflächen oder große vs. kleine Öffnungen zwischen den Inselstrukturflächen) im entfalteten Zustand gesteuert werden. Eine Änderung der Flächendichte des Geräts, um den Blutfluss zu einem Aneurysma einzuschränken, ist z. B. im Falle eines Flow diverter-Stents wünschenswert. Darüber hinaus könnten nicht-invasive Messungen medizinischer Daten, wie z. B. des Blutdrucks, drahtlos an den Arzt übertragen werden, wenn die entsprechenden Sensorelemente auf den nur geringe Spannungen aufweisenden Inselstrukturflächen funktionalisiert werden. Darüber hinaus können diese Inselstrukturflächen mit Röntgenmarkern versehen werden, die eine Drehung des Stents oder der Vorrichtung erkennen, so dass die Vorrichtungen in einer bestimmten Position mit einem bestimmten Winkel eingesetzt werden können, so dass die Funktionsinseln leicht dort platziert werden können, wo sie benötigt werden.

Die großen Zugspannungen, die mit dem superelastischen Effekt verbunden sind, ermöglichen es auxetischen Formgedächtnismaterialien, eine extrem hohe Festigkeit, eine hohe Arbeitsleistung und eine hohe Betätigungsdichte zu bieten. Es wurde bereits gezeigt, dass auxetische Stentstrukturen höhere 10-mal höhere Radialkräfte am Stentumfang aufweisen als aktuelle Stentdesigns. Dies bedeutet, dass dünnere SMA-Vorrichtungen hergestellt werden können, die sich leichter quetschen, manövrieren und im Mikrokatheter einsetzen lassen.

Die Kombination aus Flexibilität und Festigkeit, die die auxetischen Stents aus Formgedächtnislegierungen bieten, könnte auch für andere medizinische Anwendungen nützlich sein, die eine vorübergehende oder dauerhafte Öffnung eines engen Durchgangs erfordern. Dazu gehören Stents, die größere radiale Kräfte als die für das Gehirn erforderlichen benötigen, um beispielsweise in der Speiseröhre, der Harnröhre und der Prostata eingesetzt zu werden.

Aufgrund der oben genannten Eigenschaften von auxetischen Formgedächtnismaterialien eignet sich diese Erfindung für die Funktionalisierung der Außenseite von chirurgischen Geräten oder für andere temporäre medizinische Implantate. So könnte beispielsweise ein flexibles / dehnbares 3D-Implantat mit integrierten CO₂ / O₂-Sensoren, Kameras, LEDs und Drucksensoren ein nützliches Hilfsmittel für Operationen/Verfahren sein, bei denen Schläuche in die Atemwege des Patienten eingeführt werden wie von Ullah, Ramzan, et al. in "Real-Time Optical Monitoring of Endotracheal Tube Displacement." Biosensors 10.11 (2020): 174 beschrieben. Würde ein solches Gerät beispielsweise um die Außenseite eines Intubationstubus gewickelt, könnte es Ärzten / Atemtherapeuten helfen, den Intubationstubus schneller und präziser einzuführen. Die Kameras / LEDs helfen, die richtige Stelle in der Luftröhre für die Platzierung zu finden (2 cm - 5 cm über der Carina). Die CO₂ / O₂-Sensoren, die an der Außenseite des Intubationstubus (d. h. direkt an (oder über) der Manschette) integriert sind, könnten dazu beitragen, ein Entweichen von Luft zu erkennen (was auf eine Fehlplatzierung des Geräts entweder in der Lunge oder in der Speiseröhre hinweist). Die derzeitige Methode zur Erkennung der korrekten Platzierung des Tubus beruht auf Röntgenbildern. Integrierte Drucksensoren in der Manschette könnten dem Arzt helfen, den Druck zu überwachen, den er auf den Patienten ausübt, während er die Manschette (den Ballon) aufpumpt, um den Intubationstubus zu fixieren. Dies ist nur ein Beispiel dafür, wie ein auxetisches medizinisches SMA-Implantat in Verbindung mit bestehenden chirurgischen Instrumenten verwendet werden könnte.

Wie die bereits erwähnten Probleme bei der Platzierung von Stents erfordert auch die Bestätigung der Platzierung von Intubationsschläuchen häufig eine Röntgenaufnahme, die sowohl zeit- als auch kostenaufwändig ist. Mit der richtigen Anordnung (Ko-Integration) mehrerer funktioneller Vorrichtungen könnte das medizinische Implantat, das in dieser Erfindung offengelegt wird, in Zukunft diesen Schritt der Bildgebung überflüssig machen.

Auch die Behandlung von Hirnaneurysmen erfordert ein miniaturisiertes medizinisches Gerät, das nichtinvasiv mit den derzeitigen endovaskulären Behandlungsmethoden, z. B. mit einem Katheter, eingesetzt werden kann.

Stents zur Flussumleitung, Coiling und gewebte Endobridges (WEBs) sind gängige Methoden zur Behandlung von Aneurysmen. Während die meisten Arten von Hirnaneurysmen mit Flow diverter-Stents behandelt werden können, gibt es derzeit keine geeigneten intrasakkulären Geräte auf Stentbasis für die Behandlung von Aneurysmen, die die Form einer geodätischen Kuppel haben. Stents können auch nicht für Aneurysmen vom Bifurkationstyp verwendet werden, da sie den Blutfluss in einer Arterie blockieren können, was zu einem Schlaganfall führen kann. Die gängigste Methode zur Behandlung dieser Art von Aneurysma besteht darin, den Raum physisch zu besetzen (z. B. mit Pt-Spulen), um den weiteren Blutfluss in das Aneurysma einzuschränken. Eine wesentliche Nebenwirkung dieser Behandlung ist jedoch, dass die Pt-Spulen eine Rauschquelle darstellen, die eine genaue Messung des Blutflusses im Aneurysma mit herkömmlichen MRT-Techniken verhindert. Das erfindungsgemäße auxetische Design ist dazu geeignet die gleichen Vorteile wie ein WEB-Gerät sowie zusätzliche Vorteile zu bieten, da das auxetische Design mehr Freiheit bei der Größenbestimmung des Bauteils durch Photolithographie ermöglicht als ein herkömmliches Netzdesign. Darüber hinaus sind die spannungsarmen Inselstrukturflächen der erfindungsgemäßen auxetischen Struktur die einzige Art von intrasakkulären medizinischen Geräten, die funktionalisiert sind und ein direktes Auslesen von Informationen in Bezug auf den Heilungsprozess ermöglichen, wie z. B. den Blutfluss im Aneurysma, das Versagen des Geräts oder die Verringerung der Reaktion des Körpers, wie z. B. Entzündungen, auf das Gerät.

Die offengelegte Erfindung ist ein einzigartiger Gerätetyp, mit dem die dargestellten Beschränkungen für die moderne Behandlung von intrasakulären Aneurysmen überwunden werden können. Die erfindungsgemäße modifizierte dehnbare auxetische Struktur zeigt eine synklastische (konforme) Biegung um kuppelförmige Kurven (wie in dem Ausführungsbeispiel in Abb. 7 gezeigt). Diese Art des Biegeverhaltens ist mit den derzeitigen Stenttechnologien extrem schwierig oder unmöglich zu erreichen. Mit der Nutzung der erfindungsgemäßen Struktur wird eine konforme Biegung um eine Kuppelform, die für intrasakkuläre Vorrichtungen erforderlich ist, ermöglicht.

Die synklastischen Biegeeigenschaften von auxetischen SMA-Implantaten könnten auch für die Verbesserung chirurgischer Instrumente von Nutzen sein. Da sich das auxetische Substrat an die Form seiner Umgebung anpassen kann, könnte es sich um die Außenseite bestehender chirurgischer Instrumente wickeln. So könnten chirurgischen Ergebnisse verbessert werden, da der Arzt seine chirurgischen Instrumente mit neuen Geräten ausstatten könnte, die bisher nicht verfügbar waren (z. B. mit Licht, Kameras, Sensoren). Durch die Integration von Sensoren auf den Inselstrukturflächen wäre es möglich, den Blutdruck bzw. den Blutfluss im Inneren eines Aneurysmas nichtinvasiv mit diesem Gerät zu überwachen. Die nichtinvasive Überwachung des Blutflusses ohne MRT-Bildgebung könnte für die Behandlung von Hirnaneurysmen revolutionär sein. Darüber hinaus wäre die Verwendung eines kuppelförmigen SMA-Geräts im Inneren des Aneurysmas sicherer für den Patienten, da die Operationszeit verkürzt und die Sicherheit des Patienten durch eine geringere Anästhesiezeit, Strahlung und Kontrastmittelexposition erhöht würde.

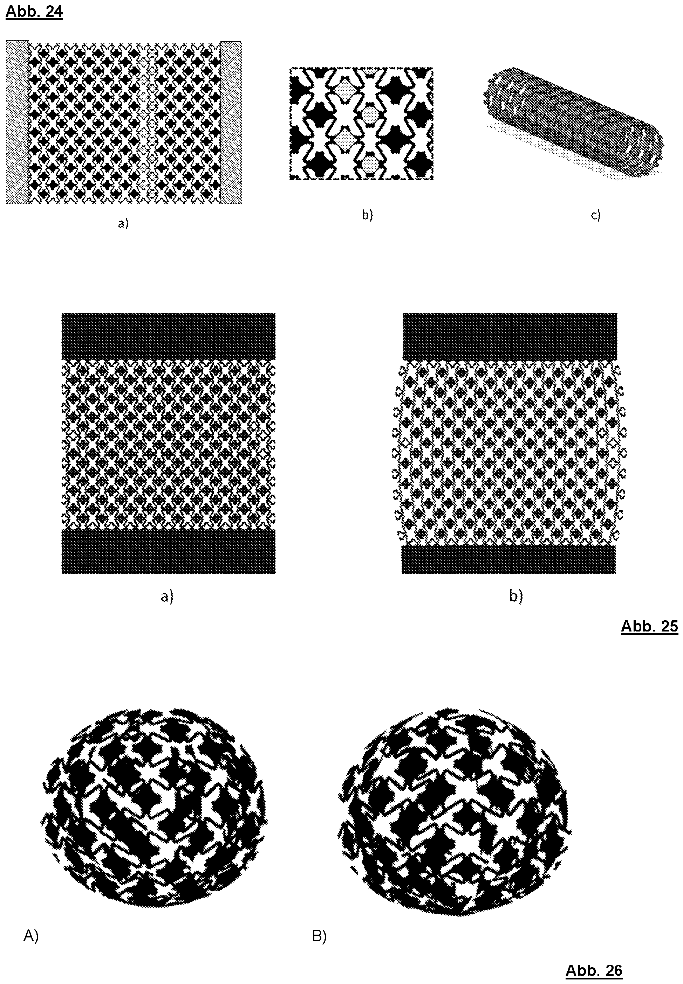

**Abb. 24** zeigt ein zweites Ausführungsbeispiel für eine "S-förmige-Inselstrukturflächen" auxetische Geometrie, so modifiziert, dass Inselstrukturflächen für die Integration von Geräten aus herkömmlichen "S-förmigen" auxetischen Strukturen zur Verfügung stehen, dargestellt als 2D-Fläche (Abb. 24A), als Ausschnitt (Abb. 24B) und als 3D intelligenter Stent (Abb. 24C). In Abb. 22B wird zudem dargestellt, dass auf dem Substrat drei verschiedene Arten von funktionalen Komponenten integriert sind. Herkömmliche "S-förmige" auxetische Strukturen sind bekannt von Meena, Kusum und Sarat Singamneni. "A new auxetic structure with significantly reduced stress concentration effects". Materials & Design 173 (2019): 107779.

In **Abb. 25** sind die Ergebnisse von Finite-Elemente-Simulationen des Ausführungsbeispiels einer "S-förmigen-Inselstrukturflächen" auxetischen Struktur aus Abb. 24 mit der Struktur im Gleichgewicht (Abb. 25A) und unter einachsiger Zugkraft (y-Richtung) (Abb. 25B) dargestellt. Es wird gezeigt, dass die modifizierte auxetische Struktur ihr auxetisches Verhalten beibehält.

**Abb. 26** zeigt die "S-förmige-Inselstrukturflächen" auxetische Geometrie, die bei der Formung zu einer 3D-Kugel eine synklastische Biegung zeigt. Dies spricht für eine mögliche Verwendung der Struktur als intrasakkuläre Aneurysma-Vorrichtung.

Als Anwendungen der Technologie insbesondere der Substratstruktur gemäß der diesseitigen Offenbarung sind zu sehen:
- funktionalisierte medizinische Geräte und Implantate (konforme Hautsensoren, Stents, perkutane Herzklappenreparatur, intrasakkuläre Geräte, dehnbarer / zusammenlegbarer Endotrachealtubus, funktioneller inferiorer Vena-Cava (IVC)-Filter);
- Raumfahrtanwendungen (verformbare Leichtbau-Raumfahrtanwendungen, d.h. Substratstruktur, die mehr Funktionalisierung auf Rovern und Raumfahrzeugen ermöglicht, entfaltbare Sonnensegel);
- Energiesammler, die direkt in Auto- und Fahrradreifen integriert sind (z. B. piezoelektrische Elemente);
- flexible / dehnbare LEDS, Displays und Kameras (einzelne Pixel/Sensoren auf den Inselstrukturflächen integriert);
- allgemeine Strukturen für tragbare und dehnbare Elektronik;
- Dehnbare Elektroden für weiche Aktuatoren;
- funktionalisierte Transportmittel (z. B. Flugzeuge für den Innen- und Außenbereich, Raumschiffe, Boote, Heißluftballons);
- Funktionalisierte Outdoor-Ausrüstung (z. B. solarbeheizter Schlafsack und Zelt);
- Aufprallresistente Energieabsorber für Fahrzeuge;
- Architektonische Gebäude (Strukturen können sich wahrscheinlich um jede Form biegen);
- Auxetische Kleidung und funktionalisierte Kleidungsstücke (z. B. Kompressionssocken, -hemden, -hosen, -schuhe, Rucksäcke, Tourniquets);
- Stoßfeste militärische Ausrüstung (z. B. kugelsichere Weste).

Die hier offenbarten Strukturen können in denselben Anwendungen eingesetzt werden, die auch für herkömmliche auxetische Strukturen verwendet werden. Insbesondere sind die Einsatzgebiete:
- Biomedizin: Arteriendilatator / Stent, medikamentenfreisetzender Stent, Stent gestütztes Coiling, intelligenter Wundverband, Tissue Engineering (d. h. künstliche Haut), künstliche Blutgefäße, Wunddruckpolster, chirurgische Geräte;
- Luft- und Raumfahrt: Schaufeln für Gasturbinentriebwerke, Wärmeschutz, Flugzeugnasen, Flügelverkleidungen, Schallschwingungsnieten;
- Automotive: Stoßfänger, Kissen, thermischer Schutz, Schwingungsdämpfer;
- Sensoren / Aktoren: Wellenausbreitung, Schwingungs-/ Dämpfungsstrukturen, intelligente Dehnungssensoren, Hydrofon, piezoelektrische Bauteile;
- Militär / Verteidigung/Sport: Aufprall-/Energie-absorbierende militärische Ausrüstung mit integrierten Sensorkomponenten (z.B. kugelsichere Weste, Helm, Knieschützer, usw.).

Wird die erfindungsgemäße auxetische Steg- oder Feldstruktur für funktionalisierte medizinische Geräte und Implantate eingesetzt, so müssen sie extremen Anforderungen standhalten können. Biosensoren, auch wearable Devices genannt, beispielsweise die direkt auf der Haut oder einem hautnah getragenen Substrat integriert werden können, müssen sich den extremen Bewegungen und den daraus resultierenden Änderungen des Krümmungsradius des Körpers anpassen können. Dehnbare Geräte können unter Verwendung von Serpentinenstrukturen, Netzstrukturen und Inselbrückenstrukturen hergestellt werden. Wenn die hier gezeigten auxetischen Strukturen aus Formgedächtnislegierungen hergestellt werden, so weisen sie viele Vorteile gegenüber den im Stand der Technik bekannten dehnbaren Designs für dehnbare Elektronik auf. Diesseits wird eine Plattform für ein extrem nachgiebiges und verformbares Substrat aufgezeigt, um die Entwicklung neuartiger dehnbarer Dünnfilm-Bauelemente zu ermöglichen. Auxetische Substrate aus einer Formgedächtnislegierung bieten überlegene mechanische Eigenschaften gegenüber dem Stand der Technik für flexible / dehnbare Designs, die auf Kupfer-Serpentinen in Insel-Brücken-Konfigurationen basieren. Formgedächtnislegierungen haben gegenüber Kupfer überlegene mechanische Eigenschaften, die es ihnen ermöglichen, sich von extrem angelegten großen Dehnungsbeträgen elastisch zu erholen (theoretisch bis zu > 8 % elastisch).

Es gibt viele Anwendungen von Formgedächtnis- / Piezoelektrischen Kompositen für verschiedene Energy Harvester und Mikroaktuator-Anwendungen. Es können beispielsweise empfindliche magnetoelektrische Sensoren, die auf piezoelektrischen und magnetostriktiven Materialien basieren, auf Formgedächtnislegierungen integriert werden. Miniaturisierte magnetoelektrische Komposite werden für Anwendungen wie bio-magnetische Sensoren, Antennen, Energy Harvester und akustische Oberflächenwellensensoren entsprechend benötigt. Es können prinzipiell alle auxetischen Sensorkonzepte durch die Bewegung des Körpers oder des Blutflusses selbst mit Energie versorgt werden, wenn sie mit einem piezoelektrischen (oder magnetoelektrischen) Energy Harvester gepaart werden. Die selektive Herstellung von AIN-Komponenten auf den spannungsarmen Inseln kann die Entwicklung empfindlicher magnetoelektrischer Sensoren für die biomagnetische Erfassung empfindlicher Signale ermöglichen, die von Herz und Gehirn kommen. Solche magnetoelektrischen Sensoren wären auch für Anwendungen wie die Tiefenhirnstimulation nützlich.

Die einzigartigen Eigenschaften der Formgedächtnislegierung können die mechanische Festigkeit / Robustheit einer dehnbaren Schaltung erhöhen und gleichzeitig die Lebensdauer des Geräts / Devices verlängern. Da SMAs im Vergleich zu traditionellen Metallen wie Kupfer deutlich größere intrinsische Dehnungen aufweisen, können enorme globale Dehnungen durch SMA-archimedische Spiralverbindungen und / oder selbstähnliche fraktale Designverbindungen erreicht werden. Dies ermöglicht Strukturen mit größerer Flächendichte, in die mehr aktive Schaltungskomponenten integriert werden können, wodurch dehnbare Devices mit höherer Effizienz möglich werden. Dank einer Struktur mit höherer Flächendichte können auch komplexere integrierte Schaltungen realisiert werden. Durch die Entwicklung der Zusammensetzung und die richtige Wärmebehandlung können Formgedächtnislegierungen auf Nickel-Titan-Basis, beispielsweise TiNiCu oder TiNiCuCo, so gestaltet werden, dass die Phasenumwandlung für bis zu 10 Millionen Zyklen reversibel ist. Daher wäre die Implementierung von auxetischen Substraten aus Formgedächtnislegierungen äußerst nützlich, um neue tragbare und verformbare elektronische Anwendungen zu ermöglichen.

Die auxetische Struktur verbessert die Anpassungsfähigkeit des elektronischen Geräts an die Haut oder das tragbare Substrat, wodurch ein vorzeitiges Versagen aufgrund von Delaminationsproblemen verhindert wird. Ein besserer Kontakt zur Haut ermöglicht besser funktionierende Sensoren, die empfindliche bio-magnetische Signale messen. Es sind entsprechende tragbare Sensoren für Magnetoenzephalographie (MEG), Magnetokardiographie (MCG), Elektroenzephalographie (EEG) und Elektrokardiogramm (EKG) MCG-Sensoren möglich, indem aktive Sensorkomponenten auf den Inselstrukturflächen der auxetischen Struktur hergestellt werden. Ultraschall-Sensoren, die in die Struktur integriert sind, würden es dem tragbaren Gerät auch ermöglichen, unter die Haut zu sehen. Diese Arten von Designs sind zudem auch für eine elektronische Haut, weiche Elektronik, Exoskelette und andere Soft-Robotik-Anwendungen vereint, sinnvoll und bevorzugt zu verwenden. Wenn die Anwendung es erfordert, ist es zudem möglich, Inselstrukturflächen elektrisch voneinander zu isolieren. Ebenso könnten Inselstrukturflächen insbesondere bevorzugt eine gemeinsame Masseelektrode durch das SMA teilen.

Es wird nicht auf jeder der auxetischen Inselstrukturflächen ein aktives elektronisches Bauelement erfordert. Außerdem muss das elektronische Bauelement nicht speziell ein Halbleiterbauelement sein. Es könnte beispielsweise eine implantierbare Struktur ausgebildet werden, die einen Sensor auf einer Inselstrukturfläche, einen Aktor auf einer anderen Inselstrukturfläche und einen Transistor (oder jede andere Art von elektronischem Bauelement) auf einer weiteren Inselstrukturfläche aufweist. Ein wesentlicher Vorteil kann zudem auch darin gesehen werden, dass die geringe Belastung auf den einzelnen Inselstrukturflächen im Wesentlichen jede Art von aktivem oder passivem Gerät erlaubt und diese elektrisch über mehrere Inselstrukturflächen miteinander verbunden sind, was wiederum eine große Fläche ermöglicht, die für entsprechende Komponenten benutzt werden kann. Darüber hinaus könnten viele andere Arten von Materialien und Geräten jetzt "dehnbar" werden (d.h. das Material muss kein Halbleiter sein, wie es bspw. in der Druckschrift US 2020/144 431 A1 der Fall ist). Im Wesentlichen kann jede Art von Bauteil in seiner ursprünglichen, traditionellen, starren Struktur bleiben.

Nachfolgend sind die Bezugszeichen aufgeführt, die in den Figuren verwendet werden:
- 1: Inselstrukturfläche einer SMA-auxetischen Struktur
- 11: erste Inselstrukturfläche einer SMA-auxetischen Struktur
- 12: zweite Inselstrukturfläche einer SMA-auxetischen Struktur
- 13: Verbindung einer SMA-auxetischen Struktur
- 2: elektrisch isolierendes Material
- 3: erste Elektrode
- 41: erste Elektronikkomponente
- 42: zweite Elektronikkomponente
- 43: dritte Elektronikkomponente
- 5: biokompatibler Einschluss
- 6: ergänzende Elektrode
- 7: Substrat

## Patentansprüche

1. Auxetische Steg- oder Feldstruktur aufweisend:
- Inselstrukturflächen und
- Verbindungen zwischen den einzelnen Inselstrukturflächen,
wobei
- offene Räume zwischen den einzelnen Inselstrukturflächen vorliegen und
- die Verbindungen mit den Inselstrukturflächen eine Steg- oder Feldstruktur ausbilden; wobei
- die Verbindungen dehnbar sind und als archimedische Spiralverbindungen und / oder selbstähnliche fraktale Designverbindungen ausgestaltet sind;
- die Verbindungen die Inselstrukturflächen an derselben Stelle und in demselben Winkel schneiden wie die vorherigen starren Verbinder das auxetische Gerüst;
- die Inselstrukturflächen unter Krafteinwirkung nicht oder nur geringfügig verbiegbar sind,
- wobei die Spannungen auf den Inselstrukturflächen mindestens eine Größenordnung unter denen der dehnbaren Verbindung liegen
und / oder
- in deren Größe nicht oder nur unwesentlich veränderlich sind
und
- die Poisson-Zahl der Struktur unter uniaxialer Verformung negativ ist.

2. Auxetische Steg- oder Feldstruktur nach Anspruch 1,
**dadurch gekennzeichnet, dass**
die einzelnen Inselstrukturflächen und / oder die einzelnen Verbindungen in Größe und Gestalt variierend ausgebildet sind.

3. Auxetische Steg- oder Feldstruktur nach Anspruch 1 oder 2,
**dadurch gekennzeichnet, dass**
wenigstens abschnittsweise über die auxetische Steg- oder Feldstruktur Bereiche gleich und / oder periodisch ausgebildet sind.

4. Auxetische Steg- oder Feldstruktur nach einem der vorangehenden Ansprüche,
**dadurch gekennzeichnet, dass**
die selbstähnlichen fraktalen Designverbindungen Koch'sche Linien, Peano'sche Linien, Hilbert'sche Linien, Moore'sche Schleifen, Vicsek'sche Schleifen und verzweigte Netze umfassen.

5. Auxetische Steg- oder Feldstruktur nach einem der vorangehenden Ansprüche,
wobei die Auxetische Steg- oder Feldstruktur planar, 2D hergestellt wird und nachfolgend an 3D-Oberflächen angepasst wird.

6. Auxetische Steg- oder Feldstruktur nach einem der vorangehenden Ansprüche,
**dadurch gekennzeichnet, dass**
wenigstens einzelne Inselstrukturflächen zur Aufnahme von Elektronik oder einzelne mit Elektronik bestückte Inselstrukturflächen vorgesehen sind.

7. Auxetische Steg- oder Feldstruktur nach dem vorangehenden Anspruch,
**dadurch gekennzeichnet, dass**
die auf den Inselstrukturflächen befindliche Elektronik über zumindest eine von der auxetischen Steg- oder Feldstruktur elektrisch isolierten Leiterbahn auf wenigstens einer Verbindung der auxetischen Steg- oder Feldstruktur miteinander elektrisch verbunden ist.

8. Verwendung der auxetischen Steg- oder Feldstruktur gemäß einem der vorangehenden Ansprüche als implantierbare Struktur aus einem biokompatiblen Material,
wobei das biokompatible Material eine metallische Formgedächtnislegierung ist und die auxetische Steg- oder Feldstruktur metallisch ausgebildet ist.

9. Verwendung der auxetischen Steg- oder Feldstruktur nach dem vorangehenden Anspruch,
**dadurch gekennzeichnet, dass**
die implantierbare Struktur eine selbstexpandierende Implantatform mit einer Zylinder- und / oder Kugel- und / oder Halbkugel- und / oder Röhren- und / oder gekrümmten Röhrenstruktur wenigstens abschnittsweise aufweist.

10. Verwendung der auxetischen Steg- oder Feldstruktur nach einem der zwei vorangehenden Ansprüche,
**dadurch gekennzeichnet, dass**
die auxetische Steg- oder Feldstruktur als gemeinsame Masseelektrode ausgebildet vorgesehen ist.

## Claims

1. Auxetic web structure or field structure comprising:
- island structure areas and
- interconnections between the individual island structure areas,
wherein
- there are open spaces between the individual island structure areas, and
- the connections with the island structure areas form a web structure or field structure; wherein
- the connections are extensible and are configured as Archimedean spiral connections and/or self-similar fractal design connections;
- the connections intersect the island structure areas at the same location and at the same angle as the previous rigid connectors intersect the auxetic framework;
- the island structure areas are not bendable or are only slightly bendable when subjected to forces,
- wherein the stresses on the island structure areas are at least one order of magnitude below those of the extensible connection
and / or
- are not or only insignificantly variable in their magnitude
and
- the Poisson's ratio of the structure under uniaxial deformation is negative.

2. Auxetic web structure or field structure according to claim 1,
wherein
the individual island structure areas and/or the individual connections are designed to vary in size and shape.

3. Auxetic web structure or field structure according to claim 1 or 2,
wherein
at least in sections over the auxetic web structure or field structure, areas are formed identically and/or periodically.

4. Auxetic web structure or field structure according to one of the preceding claims,
wherein
the self-similar fractal design connections comprise Koch's lines, Peano's lines, Hilbert's lines, Moore's loops, Vicsek's loops and branched meshes.

5. Auxetic web structure or field structure according to any one of the preceding claims,
wherein
the auxetic web structure or field structure is planar, 2D fabricated and subsequently adapted to 3D surfaces.

6. Auxetic web structure or field structure according to any one of the preceding claims,
wherein
at least individual island structure areas for receiving electronics or individual island structure areas equipped with electronics are provided.

7. Auxetic web structure or field structure according to the preceding claim,
wherein
the electronics located on the island structure areas are electrically connected to each other via at least one conductor track electrically insulated from the auxetic web structure or field structure on at least one connection of the auxetic web structure or field structure.

8. Use of the auxetic web structure or field structure according to any one of the preceding claims as an implantable structure made of a biocompatible material,
wherein
the biocompatible material is a metallic shape memory alloy and the auxetic web structure or field structure is metallic.

9. Use of the auxetic web structure or field structure according to the preceding claim,
wherein
the implantable structure has a self-expanding implant shape with a cylindrical and/or spherical and/or hemispherical and/or tubular and/or curved tubular structure at least in sections.

10. Use of the auxetic web structure or field structure according to one of the two preceding claims,
wherein
the auxetic web structure or field structure is provided in the form of a common ground electrode.

## Revendications

1. Structure auxétique de bande ou de champ comprenant
- des zones de structure en îlots et
- des interconnexions entre les différentes zones de structure en îlots,
dans laquelle
- il y a des espaces ouverts entre les différentes zones de structure en îlots, et
- les connexions avec les zones de structure en îlots forment une structure de bande ou de champ;
dans lequel
- les connexions sont extensibles et configurées comme des connexions en spirale d'Archimède et/ou des connexions fractales auto-similaires;
- les connexions coupent les zones de structure en îlots au même endroit et au même angle que les connecteurs rigides précédents coupent la structure auxétique;
- les zones de structure en îlots ne sont pas ou peu déformables sous l'effet des forces,
- dans laquelle les contraintes sur les zones de structure en îlots sont inférieures d'au moins un ordre de grandeur à celles de la connexion extensible
et / ou
- ne sont pas ou peu variables dans leur dimension
et
- le coefficient de Poisson de la structure sous déformation uniaxiale est négatif.

2. Structure auxétique de bande ou de champ selon la revendication 1,
**caractérisée en ce que**
les zones individuelles de structure en îlots et/ou les connexions individuelles sont conçues pour varier en taille et en forme.

3. Structure auxétique de bande ou de champ selon la revendication 1 ou 2,
**caractérisée en ce qu'**
au moins par sections sur la structure auxétique de la bande ou du champ, les zones sont formées de manière identique et/ou périodique.

4. Structure auxétique de bande ou de champ selon l'une des revendications précédentes,
**caractérisée en ce que**
les connexions fractales autosimilaires comprennent les lignes de Koch, les lignes de Peano, les lignes de Hilbert, les boucles de Moore, les boucles de Vicsek et les mailles ramifiées.

5. Structure auxétique de bande ou de champ selon l'une quelconque des revendications précédentes,
**caractérisée en ce que**
la structure auxétique de la bande ou du champ est planaire, fabriquée en 2D et adaptée ultérieurement à des surfaces en 3D.

6. Structure auxétique de bande ou de champ selon l'une quelconque des revendications précédentes,
**caractérisée en ce qu'**
au moins des zones de structure en îlots individuelles destinées à recevoir de l'électronique ou des zones de structure en îlots individuelles équipées d'électronique sont prévues.

7. Structure auxétique de bande ou de champ selon la revendication précédente,
**caractérisée en ce que**
l'électronique située sur les zones de structure en îlots est connectée électriquement entre elles par l'intermédiaire d'au moins une piste conductrice isolée électriquement de la structure auxétique de bande ou de champ sur au moins une connexion de la structure auxétique de bande ou de champ.

8. Utilisation de la structure auxétique de bande ou de champ selon l'une quelconque des revendications précédentes comme structure implantable en matériau biocompatible,
**caractérisée en ce que**
le matériau biocompatible est un alliage métallique à mémoire de forme et la structure de bande auxétique ou la structure de champ est métallique.

9. Utilisation de la structure auxétique de bande ou de champ selon la revendication précédente,
**caractérisée en ce que**
la structure implantable a une forme d'implant auto-expansif avec une structure cylindrique et/ou sphérique et/ou hémisphérique et/ou tubulaire et/ou tubulaire incurvée au moins en sections.

10. Utilisation de la structure auxétique de bande ou de champ selon l'une des deux revendications précédentes,
**caractérisée en ce que**
la structure de bande auxétique ou la structure de champ se présente sous la forme d'une électrode de masse commune.
